# EUROPEAN PATENT APPLICATION

(11) **EP 4 019 633 A1**
(43) Date of publication of application: **29.06.2022**
(21) Application number: 20217048.6
(22) Date of filing: 23.12.2020
(51) Int. Cl.: C12N 15/113, B82Y 5/00, C07K 16/30, A61K 39/44, C12N 15/115

(54) **CONDITIONAL CELL CONNECTORS**

(71) Applicant: Technische Universität München, 80333 München (DE)
(72) Inventor: DIETZ, Hendrik, 85540 Haar (DE); FUNKE, Jonas Jörg, 85748 Garching bei München (DE); KICK, Benjamin, 85368 Moosburg (DE); WAGENBAUER, Klaus, 85748 Garching bei München (DE)
(74) Representative: Engelhard, Markus

(57) **Abstract**

The present invention relates to a nucleic acid nanostructure comprising a first surface and a second surface, wherein said first surface and said second surface are located at opposing sides of said nanostructure, wherein said first surface comprises at least a first targeting agent and said second surface comprises at least a second targeting agent and at least a third targeting agent. The present invention further relates to a composition comprising a nucleic acid nanostructure. The invention also relates to a nanostructure and a composition for use in medicine, and to a nanostructure and a composition for use in a method of preventing or treating a disease. Furthermore, the present invention relates to a method of preparing a nanostructure and to a use of a nanostructure for binding first target and the second target.

## Description

### FIELD OF THE INVENTION

The present invention relates to a nucleic acid nanostructure comprising a first surface and a second surface, wherein said first surface and said second surface are located at opposing sides of said nanostructure, wherein said first surface comprises at least a first targeting agent and said second surface comprises at least a second targeting agent and at least a third targeting agent. The present invention further relates to a composition comprising a nucleic acid nanostructure. The invention also relates to a nanostructure and a composition for use in medicine, and to a nanostructure and a composition for use in a method of preventing or treating a disease. Furthermore, the present invention relates to a method of preparing a nanostructure and to a use of a nanostructure for binding first target and the second target.

### BACKGROUND OF THE INVENTION

Immune-cell engaging therapy has great potential for the treatment of cancer. A targeted approach considers bispecific T-cell engaging antibodies. These compounds target the cancer cell on the one side and T cells on the other. For instance, the bispecific antibody Blinatumomab (anti-CD3 x anti-CD 19 BiTE) was approved by the European Medicines Agency based on its efficacy for treating refractory acute lymphocytic leukemia (ALL). It recruits CD3-positive cytotoxic T cells (immune cells) to CD19-positive B cells (target cells) and induces the formation of a cytolytic immunological synapse between the T cell and the target cell [1]. The formation of the immunological synapse promotes effective target-cell killing by cytotoxic T cells through the release of pore-forming protein perforin and the apoptosis-inducing granzyme B. Importantly, the immunological synapse can only be induced if both cells are held in close proximity [2, 3].

T-cell engaging antibodies are therefore designed for small paratope-to-paratope distances. In particular, the seven-nanometer paratope-to-paratope of Blinatumomab contributes to its high target-cell-killing efficacy. One major challenge faced by bispecific antibody therapies is, however, balancing desired on-tumor T-cell-mediated target-cell killing with un-desired off-tumor activity. Because the target antigens are often not exclusively present on tumor cells but are also present in healthy tissue, "on-target off-tumor" activity leads to killing of healthy tissue and immune overstimulation which may be toxic. For the ALL indication, where a tumor specific antigen (CD19) exists, Blinatumomab and similar approaches are highly effective with limited side effects. On the contrary, for indications, where no single tumor-specific antigen can be identified, these approaches will usually fail because of strong on-target off-tumor side effects [4].

Recently, new methods for manufacturing nanostructures have been developed, such as manufacturing nucleic acid nanostructures, e.g. "DNA origami", as disclosed in US 7,842,793 B3. DNA origami are nucleic acid nanostructures that can be designed to have a predetermined structure. DNA origami uses DNA molecules for the production of three dimensional structures on the nanometer scale. WO 2014/170898 A1 and WO 2012061719 relate to dynamic two-part DNA-origami devices that were equipped with a DNA-aptamer-latch mechanism to conditionally present a cargo (e.g. antibody) at a target's cell surface. Such dynamic two-part DNA-origami devices rely on multi-domain objects with flexible parts and the conformational state depends on the local environment (e.g. ionic strength). A nanostructure which is robust and more inert to the local environment would be highly advantageous. Furthermore, nanostructures may be protected against digestion from nucleases or against disassembly by low-ionic-strength environments using, for example, PEG-oligolysine coating as described in ["Oligolysine-based coating protects DNA nanostructures from low-salt denaturation and nuclease degradation", Ponnuswamy et al., Nat Commun 2017; WO2015070080]. These coatings and stabilization methods may interfere with the conformational states and configurations of flexible multi-domain objects. Conversely, a rigid single-domain nanostructure with one configuration would be highly advantageous because it may be stabilized using said stabilization methods without an impairment of its conditional cell-recruiting function. Furthermore, a nanostructure which allows to target more than one cell type or antigen would be highly advantageous. Furthermore, a nanostructure which promotes cell interactions only if a particular antigen pattern is recognized would be highly advantageous.

Accordingly, there is a need for providing enhanced targeting means, such as nanostructures capable of targeting more than one cell type and/or of targeting more than one cell-surface molecule and/or of targeting a combination of cell-surface molecules. Furthermore, there is a need to provide means for specific targeting cellular targets, such as immune cells and/or tumor cells. Particularly, there is a need for means that allow bringing cellular targets into proximity. Furthermore, there is a need for means that allow activating cellular targets, such as cytotoxic T cells, preferably by simultaneously bringing at least one further cellular target into proximity of the activated cellular target. There is also the need for means that enable the formation of an immunological synapse. Additionally, there is a need for means that allow to selectively and/or specifically target one or more targets. Additionally, there is a need for means that allow to selectively and/or specifically target cells that present a combination or pattern of cell-surface molecules (e.g. antigens).

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a nucleic acid nanostructure comprising a first surface and a second surface, wherein said first surface and said second surface are located at opposing sides of said nanostructure, wherein said first surface comprises at least a first targeting agent and said second surface comprises at least a second targeting agent and at least a third targeting agent.

In one embodiment, said nanostructure has a longitudinal axis and a transverse axis, wherein said first surface and said second surface are located at opposing sides along said longitudinal axis.

In one embodiment, the nanostructure has a maximum longitudinal extension along said longitudinal axis which is larger than a maximum transverse extension along said transverse axis, preferably larger than a synaptic distance, more preferably larger than at least 10 nm, e.g. at least 25 nm; and/or
the nanostructure has a maximum transverse extension along said transverse axis which is smaller than a maximum longitudinal extension along said longitudinal axis, preferably smaller than a synaptic distance, more preferably smaller than 25 nm, e.g. ≤ 10 nm.

In one embodiment, the nanostructure has a maximum longitudinal extension along said longitudinal axis which is larger than a maximum transverse extension along said transverse axis, preferably larger than a synaptic distance, more preferably larger than at least 10 nm, e.g. at least 25 nm.

In one embodiment, the nanostructure has a maximum transverse extension along said transverse axis which is smaller than a maximum longitudinal extension along said longitudinal axis, preferably smaller than a synaptic distance, more preferably smaller than 25 nm, e.g. ≤ 10 nm.

In one embodiment, the first targeting agent is configured to bind to a first target molecule on a first target, preferably first target cell, more preferably immune cell, wherein the second targeting agent is configured to bind to a second target molecule on a second target, preferably second target cell, more preferably diseased cell, and wherein the third targeting agent is configured to bind to a third target molecule on said second target, preferably second target cell, more preferably diseased cell.

In one embodiment, the first targeting agent, the second targeting agent, and the third targeting agent are independently selected from an antibody or antigen-binding fragment thereof, an antigen-binding peptide, a Fab fragment, a F(ab')₂ fragment, a Fv fragment, a diabody, a single chain Fv fragment, a (scFv)₂, a tetrabody, a triabody, a disulfide bond-stabilized Fv (dsFv), a Fc domain, an engineered Fc domain, a DNA aptamer, a RNA aptamer, a peptide nucleic acid (PNA), a polypeptide, a peptide, a glycoprotein, a peptidomimetic, an anticalin, an Affilin, an Affimer, an Affitin, an Alphabody, a nanobody, DARPin, a receptor ligand, a receptor or fragment thereof, and a receptor domain, preferably independently selected from an antibody or antigen-binding fragment thereof, an antigen-binding peptide, a Fab fragment, a F(ab')₂ fragment, a Fv fragment, a diabody, a single chain Fv fragment, a (scFv)₂, a tetrabody, a triabody, a disulfide bond-stabilized Fv (dsFv), a Fc domain, an engineered Fc domain, a polypeptide, a peptide, a glycoprotein, a peptidomimetic, an anticalin, an Affilin, an Affimer, an Affitin, an Alphabody, a nanobody, DARPin, a receptor ligand, a receptor or fragment thereof, and a receptor domain.

In one embodiment, the second targeting agent and the third targeting agent are spaced at a distance from each other, preferably at a distance along the longitudinal axis larger than a synaptic distance, for example of at least 15 nm, preferably at least 20 nm, more preferably at least 40 nm; and/or
said nanostructure is a rigid nanostructure, and/or is a nanostructure without a hinge region and/or is a nanostructure having only one configuration, and/or is a nanostructure comprising or being a DNA origami, wherein said DNA origami has only one configuration; and/or
the nanostructure comprises a maximum length, and wherein the maximum length is smaller than 1000 nm, preferably smaller than 500 nm, such as 100 nm, wherein, preferably, said maximum length is a length of the maximum longitudinal extension.

In one embodiment, the second targeting agent and the third targeting agent are spaced at a distance from each other, preferably at a distance along the longitudinal axis larger than a synaptic distance, for example of at least 15 nm, preferably at least 20 nm, more preferably at least 40 nm.

In one embodiment, said nanostructure is a rigid nanostructure, and/or is a nanostructure without a hinge region and/or is a nanostructure having only one configuration, and/or is a nanostructure comprising or being a DNA origami, wherein said DNA origami has only one configuration.

In one embodiment, the nanostructure comprises a maximum length, and wherein the maximum length is smaller than 1000 nm, preferably smaller than 500 nm, such as 100 nm, wherein, preferably, said maximum length is a length of the maximum longitudinal extension.

In one embodiment, said first target cell is an immune cell, preferably selected from the group consisting of lymphocytes, e.g. T cells, cytotoxic T cells, B cells, natural killer cells, natural killer T cells, CAR-T cells, monocytes, macrophages, and neutrophils, more preferably selected from the group consisting of lymphocytes, e.g. T cells, cytotoxic T cells, B cells, natural killer cells, natural killer T cells, and CAR-T cells; and/or said second target cell is a diseased cell, preferably selected from a cancer cell, a tumor cell, a cell involved in an autoimmune response, and an infected cell, e.g. a cell infected with a virus, mycoplasma, bacterium, or parasite.

In one embodiment, said first target cell is an immune cell, preferably selected from the group consisting of lymphocytes, e.g. T cells, cytotoxic T cells, B cells, natural killer cells, natural killer T cells, CAR-T cells, monocytes, macrophages, and neutrophils, more preferably selected from the group consisting of lymphocytes, e.g. T cells, cytotoxic T cells, B cells, natural killer cells, natural killer T cells, and CAR-T cells.

In one embodiment, said second target cell is a diseased cell, preferably selected from a cancer cell, a tumor cell, a cell involved in an autoimmune response, and an infected cell, e.g. a cell infected with a virus, mycoplasma, bacterium, or parasite.

In one embodiment, said first target molecule is a surface molecule of an immune cell, preferably selected from CD3, CD3δ/ε, CD3γ/ε, TCR, TCRα, TCRβ, CD2, CD5, CD28, OX40, 4-1BB, CD16, Ly49, NKp30 (CD337), NKp44 (CD336), NKp46 (NCR1), CD3ζ (CD247), CD27, CD40, CD137, CD64, CD89, toll-like receptors (TLR), cytokine receptors, a Fc domain, an engineered Fc domain, GITR, and ICOS; and/or
said second target molecule and said third target molecule are independently selected from CD2, CD7, CD10, CD13, CD15, CD19, CD24, CD28, CD29, CD33, CD34, CD38, CD44, CD45, CD49f, CD56, CD57, CD60a, CD66/CEA, CD79a, CD117, CD123, CD138, CD140b, CD227/MUC1, CD243/MDR, CD244, CD326/EpCAM, CD340/HER2, VEGF-R, EGFR, CSPG4/MCSP, MAGs, CA125, PSMA, HLA-DR, carbonic anhydrase 9, aquaporin, PSMA, TIM3, CLL1/CLEC12A, EGFR v3, and HLA-A2, wherein, preferably, said second target molecule and said third target molecule are not identical; and/or
said second target molecule and said third target molecule are a combination of target molecules, wherein said combination is selected from CD33 and CD123, CD326/EpCAM and CD10, CD326/EpCAM and CD340/HER2, CD326/EpCAM and VEGF-R, CD326/EpCAM and EGFR, CD326/EpCAM and CD243/MDR, CSPG4/MCSP and CD326/EpCAM, CSPG4/MCSP and MAGs, CA125 and CD227/MUC1, CA125 and CD227/MUC1, CD56 and CD140b, CD56 and CD60a, EGFR and CD340/HER2, PSMA and CD340/HER2, CD15 and EGFR, CD44 and CD117, CD44 and CD326/EpCAM, CD34 and CD19, CD34 and CD79a, CD34 and CD2, CD34 and CD7, CD34 and HLA-DR, CD34 and CD13, CD34 and CD117, CD34 and CD33, CD34 and CD15, CD33 and CD19, CD33 and CD79a, CD33 and CD2, CD33 and CD7, CD33 and HLA-DR, CD33 and CD13, CD33 and CD117, CD33 and CD15, CD227/MUC1 and CD10, CD227/MUC1 and CD66/CEA, CD227/MUC1 and CD57, CD38 and CD138, CD24 and CD29, CD24 and CD49f, carbonic anhydrase 9 and aquaporin, CD19 and CD33, CD19 and CD22, CD28 and PSMA, CD227/MUC1 and EGFR, CD33 and TIM3, CD123 and TIM3, CLL1/CLEC12A and TIM3, CD244 and TIM3, CD33 and EGFR v3, CLL1/CLEC12A and EGFR v3, CD123 and EGFR v3, and CD45 and HLA-A2.

In one embodiment, said first target molecule is a surface molecule of an immune cell, preferably selected from CD3, CD3δ/ε, CD3γ/ε, TCR, TCRα, TCRβ, CD2, CD5, CD28, OX40, 4-1BB, CD16, Ly49, NKp30 (CD337), NKp44 (CD336), NKp46 (NCR1), CD3ζ (CD247), CD27, CD40, CD137, CD64, CD89, toll-like receptors (TLR), cytokine receptors, a Fc domain, an engineered Fc domain, GITR, and ICOS.

In one embodiment, said second target molecule and said third target molecule are independently selected from CD2, CD7, CD10, CD13, CD15, CD19, CD24, CD28, CD29, CD33, CD34, CD38, CD44, CD45, CD49f, CD56, CD57, CD60a, CD66/CEA, CD79a, CD117, CD123, CD138, CD140b, CD227/MUC1, CD243/MDR, CD244, CD326/EpCAM, CD340/HER2, VEGF-R, EGFR, CSPG4/MCSP, MAGs, CA125, PSMA, HLA-DR, carbonic anhydrase 9, aquaporin, PSMA, TIM3, CLL1/CLEC12A, EGFR v3, and HLA-A2, wherein, preferably, said second target molecule and said third target molecule are not identical.

In one embodiment, said second target molecule and said third target molecule are a combination of target molecules, wherein said combination is selected from CD33 and CD123, CD326/EpCAM and CD10, CD326/EpCAM and CD340/HER2, CD326/EpCAM and VEGF-R, CD326/EpCAM and EGFR, CD326/EpCAM and CD243/MDR, CSPG4/MCSP and CD326/EpCAM, CSPG4/MCSP and MAGs, CA125 and CD227/MUC1, CA125 and CD227/MUC1, CD56 and CD140b, CD56 and CD60a, EGFR and CD340/HER2, PSMA and CD340/HER2, CD15 and EGFR, CD44 and CD117, CD44 and CD326/EpCAM, CD34 and CD19, CD34 and CD79a, CD34 and CD2, CD34 and CD7, CD34 and HLA-DR, CD34 and CD13, CD34 and CD117, CD34 and CD33, CD34 and CD15, CD33 and CD19, CD33 and CD79a, CD33 and CD2, CD33 and CD7, CD33 and HLA-DR, CD33 and CD13, CD33 and CD117, CD33 and CD15, CD227/MUC1 and CD10, CD227/MUC1 and CD66/CEA, CD227/MUC1 and CD57, CD38 and CD138, CD24 and CD29, CD24 and CD49f, carbonic anhydrase 9 and aquaporin, CD19 and CD33, CD19 and CD22, CD28 and PSMA, CD227/MUC1 and EGFR, CD33 and TIM3, CD123 and TIM3, CLL1/CLEC12A and TIM3, CD244 and TIM3, CD33 and EGFR v3, CLL1/CLEC12A and EGFR v3, CD123 and EGFR v3, and CD45 and HLA-A2.

In one embodiment, said nucleic acid comprises or consists of DNA; and/or
the nanostructure comprises or is a DNA origami structure,
wherein the DNA origami structure comprises at least one scaffolding strand,
wherein the DNA origami structure further comprises a plurality of single-stranded oligonucleotide staple strands,
wherein each staple strand is at least partially complementary to at least one scaffolding strand, and
wherein each of the staple strands is configured to bind to at least one of the at least one scaffolding strand in at least one place, preferably in two or more distinct places,
wherein the at least one scaffolding strand is folded and/or arranged such that the desired nanostructure is formed.

In one embodiment, said nanostructure comprises a recess, preferably a recess oriented perpendicular to said maximum longitudinal extension,
wherein, preferably, said first targeting agent is bound to said nanostructure via said recess and/or at least a portion of said first targeting agent is located within said recess.

In one embodiment, said nanostructure further comprises an active agent, preferably drug, and/or a marker, e.g. imaging marker, wherein said active agent and/or marker is/are coupled to said nanostructure, optionally via a linker.

In a further aspect, the present invention relates to a composition, preferably pharmaceutical composition, comprising a nucleic acid nanostructure, as defined above, optionally further comprising a pharmaceutically acceptable excipient.

In a further aspect, the present invention relates to a nanostructure, as defined above, or the composition, as defined above, for use in medicine.

**In** a further aspect, the present invention relates to a nanostructure, as defined above, or a composition, as defined above, for use in a method of preventing or treating a disease selected from proliferative diseases, such as cancer, immunological disorders, e.g. autoimmune diseases, infectious disorders, e.g. viral diseases such as a human immunodeficiency virus (HIV) infection, metabolic disorders, and/or diabetes;
said method preferably comprising binding a first target cell and a second target cell using said nanostructure,
more preferably comprising inducing, promoting, stabilizing, and/or inhibiting a formation of a synapse, preferably only if the first, second, and third targeting agents have bound to their respective target molecules,
even more preferably comprising binding said first target cell and said second target cell using said nanostructure such that, as a result of said binding, said first target cell and said second target cell have a distance ≤ 25 nm, preferably ≤ 15 nm.

In a further aspect, the present invention relates to a method of preparing a nanostructure, as defined above, comprising the steps:
i) providing a nucleic acid nanostructure, preferably rigid nucleic acid nanostructure,
ii) providing a first targeting agent, a second targeting agent, and a third targeting agent, wherein each of said targeting agents is conjugated to a nucleic acid strand that is complementary to a nucleic acid strand, e.g. scaffolding strand or DNA handle strand, of said nanostructure,
iii) obtaining a nucleic acid nanostructure comprising said first targeting agent, said second targeting agent, and said third targeting agent, preferably by self-assembly of said nanostructure.

Said nucleic acid nanostructure, said rigid nucleic acid nanostructure, said first targeting agent, said second targeting agent, and said third targeting agent are as defined above.

In a further aspect, the present invention relates to a use of a nanostructure, as defined above, or composition, as defined above, for binding a first target and a second target;
preferably for inducing, promoting, stabilizing, and/or inhibiting a formation of a synapse between a first target cell and a second target cell, preferably only if the first, second, and third targeting agents have bound to their respective target molecules;
more preferably comprising binding said first target, e.g. first target cell, and said second target, e.g. second target cell, using said nanostructure such that, as a result of said binding, said first target and said second target have a distance ≤ 25 nm, preferably ≤ 15 nm.

Said nucleic acid nanostructure, said first target, said second target, said first target cell, and said second target cell are as defined above.

In a further aspect, the present invention relates to a use of a nanostructure, as defined above, or a composition, as defined above, for the manufacture of a medicament, e.g., a medicament for preventing or treating a disease selected from proliferative diseases, such as cancer, immunological disorders, e.g. autoimmune diseases, infectious disorders, e.g. viral diseases such as a human immunodeficiency virus (HIV) infection, metabolic disorders, and/or diabetes.

In a further aspect, the present invention relates to a method of preventing and/or treating a disease, preferably a disease selected from proliferative diseases, such as cancer, immunological disorders, e.g. autoimmune diseases, infectious disorders, e.g. viral diseases such as a human immunodeficiency virus (HIV) infection, metabolic disorders, and/or diabetes, comprising administering a nanostructure, as defined above, and/or a composition, as defined above, to a patient in need thereof.

In one embodiment, said administering comprises administering an effective amount of a nanostructure, as defined above, and/or a composition, as defined above, to a patient in need thereof.

### DETAILED DESCRIPTION

The present invention relies on a rigid DNA-origami platform on which targeting agents, such as antibodies, are positioned. Such rigid nucleic acid nanostructures are highly advantageous compared to complex hinge mechanisms or aptamer-latch systems or complex protein engineering, for example since such rigid nucleic acid nanostructures are more suited for in vivo use and/or their production is more cost- and/or time-efficient. The nanostructures of the present invention are highly robust, especially in in vivo conditions, where local environments may vary depending on the tissue. Advantageously, compared to nanostructures comprising hinge regions, the nanostructures of the invention do not rely on fine-tuning conformational states. Instead, the nanostructures can be efficiently used and/or produced by fine-tuning the DNA-origami size and shape. The nanostructures of the invention are also advantageous in that they can target at least two target cells, and can even be extended to target further cells and/or further targets, e.g. by targeting three different antigens. The nanostructures of the invention are further advantageous in that cells are only contacted, recognized, and/or eliminated, if at least the first, second, and third targeting agents of said nanostructure bind to their respective targets. A further advantage is that only those second targets, preferably second target cells, are contacted, recognized, and/or eliminated which comprise at least the second target molecule and the third target molecule. Accordingly, only those target cells are recognized and/or eliminated which comprise the respective target molecules. Thereby, off-binding and/or unspecific binding is reduced. The nanostructure of the invention is highly advantageous in that it is highly selective and specific, since a first target and a second target are bound to each other and/or are contacted, preferably a synapse is formed, only if at least the first target agent, the second target agent, and the third target agent are bound to their respective target molecules.

The modularity of the DNA-origami technology, particularly of the nanostructure of the invention, allows for quick and efficient adjustment to different indications and settings.

In order to make the highly effective method of directing cytotoxic T cells to target cells available for indications where no one single tumor-specific antigen can be identified, the inventors developed a multispecific T-cell engaging platform, e.g. antibody platform, using the DNA-origami method. Mono-specific target-cell and T-cell targeting agents such as antibodies are combined on this platform to form a functional bi- or multispecific object that can recruit first target cells, such as immune cells e.g. T cells, to second target cells, such as cancer cells. However, immune cell- e.g. T-cell-mediated killing is initiated only if all second target targeting agents, preferably at least the second targeting agent and the third targeting agent, are bound to their respective target molecules e.g. antigens on the surface of the target cell. Thus, diseased cell such as tumor cells can be targeted based on a complex antigen pattern (i.e. antigen combination) rather than based on a single antigen. For example, acute myelogenous leukemia (AML) cells could be identified based on a combination of AML markers, such as CD33 and CD123 antigens.

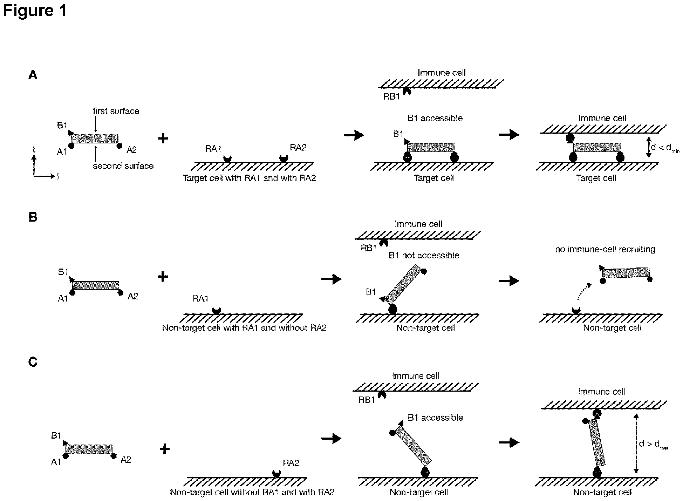

The inventors achieve this conditional killing of target cells, preferably second target cells, by mounting multiple targeting agents, e.g. antibodies, against target molecules, e.g. target-cell antigens, and at least one immune-cell binding targeting agent, preferably first targeting agent, e.g. antibody, on the platform (Figure 1). In one embodiment, the term platform relates to a nanostructure. The shape of the nanostructure e.g. DNA-origami platform is engineered such that it has at least one small dimension (e.g. smaller than 10 nm; this dimension needs to be chosen to form the desired immunological synapse, or cell-cell interaction). In one embodiment, such small dimension is referred to as a maximum transverse extension and/or maximum transverse length. The second and third targeting agents are mounted such that only if both the second and the third targeting agents are bound to their respective target molecules, e.g. target-cell antigens, on the same cell, the platform is parallel to the target-cell surface, preferably the surface of a second target cell, with the short dimension perpendicular to the cell surface (Figure 1a vs 1b and 1c). This orientation allows for an improved accessibility of the first targeting agent, e.g. immune-cell-binding antibody, and allows that the first target, e.g. cytotoxic T cell, can come in close proximity to the second target. Hence, the length of the small dimension, the targeting agents, and their attachment must be chosen such that a cytolytic immunological synapse can be formed. If not both of the second and third targeting agent, e.g. not all target-cell antibodies, are bound, the first targeting agent, e.g. immune-cell-binding antibody, is not or limited accessible (Figure 1b) or the distance between the first target e.g. T cell and the second target, e.g. a diseased target cell, is too large to initiate an immunological synapse (Figure 1c).

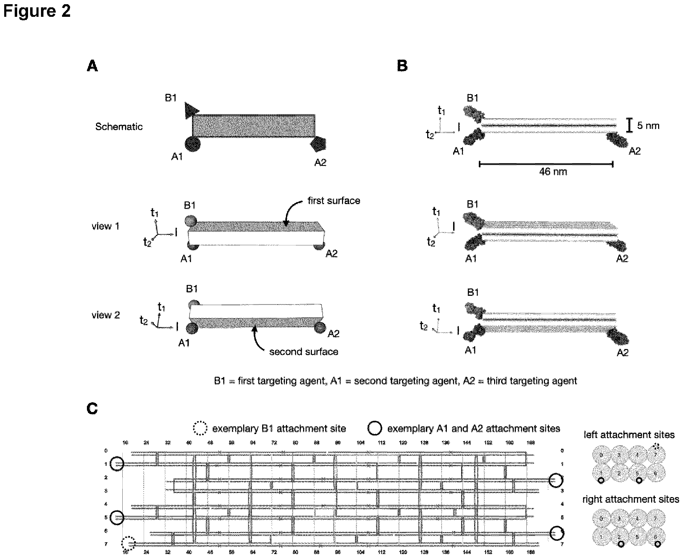

In one embodiment, such a device, preferably nanostructure, has a rod-like shape (Figure 2), and optionally a second targeting agent A1 and a third targeting agent A2, e.g. antibodies A1 and A2, against the second target, e.g. diseased target-cell, are attached on ends of said device. On one end, a first targeting agent B1 e.g. an immune-cell binding antibody B1 is attached. Only if both targeting agents A1 and A2 are bound to their respective target molecule e.g. antigen targets on the target cell, the rod will be parallel to the cell surface, and an immune cell can come in close proximity to form a cytolytic synapse.

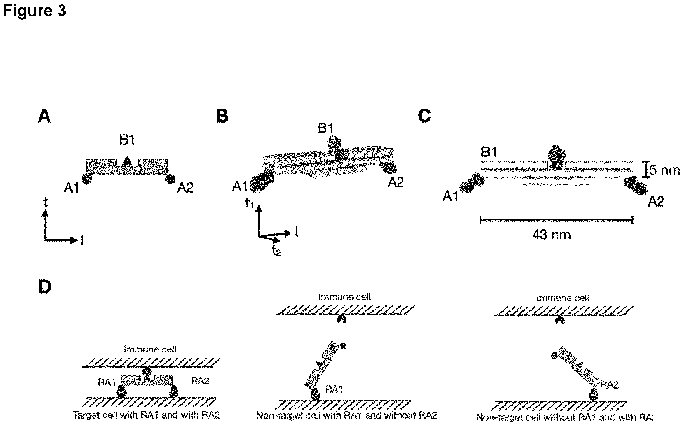

Furthermore, by forming a recess in the DNA-origami object in which a targeting agent, e.g. a first targeting agent such as the immune-cell binding antibody, is placed, the accessibility of the targeting agent e.g. immune-cell binding antibody and thus the conditional properties of the device e.g. nanostructure may be adjusted (Figure 3). In addition, this device could also be used to identify target cells based on their antigen density if the same target-cell antigens are mounted: both target-cell antibodies can only bind if the antigen density is high enough that the second target-cell antibody can bind before the first antibody detaches from the surface.

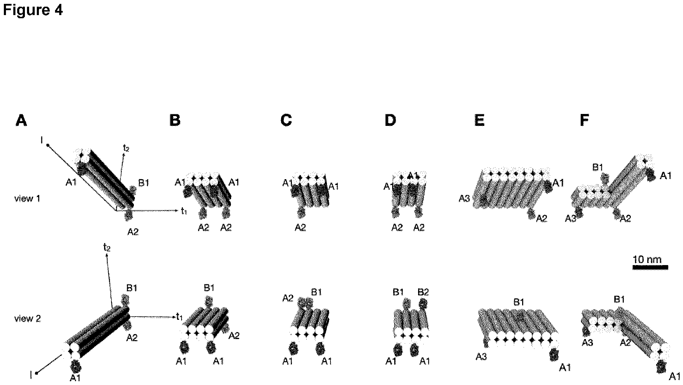

Furthermore, on which end of the rod-like platform the first targeting agent e.g. immune-cell binding antibody is placed may be chosen according to the distribution of the target antigens on target and non-target cells. For example, if antigen RA1 is also common on non-target cells, it may be advantageous to place the immune-cell recruiting antibody close to A1. This is because binding to RA1 renders B1 inaccessible such that no immune cell can bind and such that the antibody-DNA platform may not bridge two cells but detach from the cell surface to find the next potential target cell. In one embodiment, when referring to a platform, object, or device, a nanostructure is meant. The herein presented approach can be extended to recognize more than two target molecules on a second target cell, e.g. target antigens, using, for example, triangular-shaped objects (Figure 4). Furthermore, more than one immune-cell binding moiety may be placed on the platform. This could be advantageous in tumor microenvironments, where immune-cell activation is inhibited. By placing more than one immune-stimulating molecule, a local immune response may be triggered even in immune-inhibited environments. In one embodiment, a first targeting agent, and optionally a fourth or further targeting agent are immune stimulating agents.

The inventors previously constructed dynamic DNA-origami devices which consist of multiple parts and which are connected through flexible hinges to conditionally recruit cells. These nanodevices rely on engineered equilibrium states to fulfill their conditional function. Particularly, the occupancy of conformational states depends on the local environment (e.g. ionic strength, pH, protein corona). The herein presented approach is very advantageous in that it relies on rigid nanostructures, e.g. single-body antibody-carrier DNA-platforms, which are more inert and robust than flexible multi-domain objects. The nanostructures of the invention are thus highly robust against changes in the local environment (e.g. changes of pH, ionic strength, protein corona, nuclease concentration, and others). These increased stability and robustness properties may be especially important in in vivo settings or when targeting solid tumors that create tumor microenvironments different from healthy tissue.

In one embodiment, the shape of the DNA-origami platform is designed such that one dimension is smaller than the distance required for immune-synapse formation, e.g. smaller than 20 nm. In one embodiment, the antibodies are positioned in such a way that only multivalent binding, e.g. binding of each targeting agent to its respective target, leads to a parallel orientation of the platform, e.g. parallel to a surface of a target cell. In addition, the distance-dependent synapse formation is recognized.

In one embodiment, the term "targeting agent" relates to a moiety capable of binding a target on a cell surface, preferably specifically binding said target, e.g. an antigen-binding peptide. In one embodiment, a targeting agent is any of an antibody or an antigen-binding peptide thereof, for example an IgG, a Fab fragment, a single-chain Fab fragment, or a single domain antibody, a DNA and/or RNA aptamer, a peptide, a binding protein, a ligand of a cell-surface receptor (e.g. PD-L1), or a lipid, e.g. any of an antibody or antigen-binding fragment thereof, an antigen-binding peptide, a Fab fragment, a F(ab')₂ fragment, a Fv fragment, a diabody, a single chain Fv fragment, a (scFv)₂, a tetrabody, a triabody, a disulfide bond-stabilized Fv (dsFv), a DNA aptamer, a RNA aptamer, a peptide nucleic acid (PNA), a polypeptide, a peptide, a glycoprotein, a peptidomimetic, an anticalin, an Affilin, an Affimer, an Affitin, an Alphabody, a nanobody, DARPin, a receptor ligand, a receptor or fragment thereof, and a receptor domain. In one embodiment, the targeting agent is an antigen-binding peptide, such as an antibody, an antibody-like molecule, an antibody mimetic, an antigen binding derivative, or an antigen-binding fragment thereof. In one embodiment, the interaction between a targeting agent and its target is characterized by the specific binding of an antibody-antigen interaction. The person of skill is able to identify similar specific interactions, which shall also be within the scope of this invention. As an example, the specific binding of an aptamer to a molecule, or of a receptor ligand to its receptor, may also be used, or vice versa. However, a great variety of interactions may be utilized in such a nanostructure, which may be advantageous compared to the state of the art that relies on specific and limited interactions, e.g. interactions of a receptor and a ligand. Thus, owing to the wide range of interactions available, a more versatile and adjustable DNA nanostructure platform is provided compared to nanostructures disclosed in the prior art. For example, the targeting agent can be an IgG antibody, an IgG antibody fragment, an IgG antibody mimetic, an IgM antibody, an IgM antibody fragment, an IgM antibody mimetic, an IgD antibody, an IgD antibody fragment, an IgD antibody mimetic, an IgA antibody, an IgA antibody fragment, an IgA antibody mimetic, an IgE antibody, an IgE antibody fragment, or an IgE antibody mimetic.

In one embodiment, the targeting agent reversibly or irreversibly binds to its target. For example, reversibly binding molecules may be an antibody, an antibody fragment, a DNA strand, a biotin molecule, a streptavidin molecule, whereas irreversibly binding molecules may be a maleimide-thiol chemistry molecule or a click chemistry molecule. In one embodiment, a first targeting agent, a second targeting agent, and a third targeting agent are the same or different, preferably different from each other. In one embodiment, a second targeting agent and a third targeting agent are the same or different, preferably different from each other. In one embodiment, a second targeting agent and a third targeting agent target the same target molecule and/or different target molecules, preferably target different target molecules. In one embodiment, a second target molecule and a third target molecule are the same or different. In one embodiment, an exemplary first targeting agent is any of anti-CD3 epsilon (e.g. Fab), anti-CD28 (e.g. Fab), and IL2, or combinations thereof. In one embodiment, an exemplary second targeting agent is any of anti-CD 19 (e.g. Fab), anti-CD 123 (e.g. Fab), and anti-CD33 (e.g. Fab), or combinations thereof. In one embodiment, an exemplary third targeting agent is any of anti-CD19 (e.g. Fab), anti-CD123 (e.g. Fab), and anti-CD33 (e.g. Fab), or combinations thereof.
In one embodiment, an immune-cell targeting agent is any immune-cell binding molecule, protein, moiety, and/or complex, e.g. an immune-cell stimulating or inhibiting molecule, protein, moiety, and/or complex. In one embodiment, an immune-cell targeting agent, e.g. immune-cell binding antibody, is an agent that can bind, activate and/or co-stimulate T cells or natural killer cells, e.g. an anti-CD3 antibody, anti-CD28 antibody, anti-CD137 antibody, anti-CTLA-4 antibody, a TLR agonist (e.g. TLR-7/8, TLR-3, TLR-4, TLR-9), a cytokine (e.g. IL-2), a check-point ligand or inhibitor (e.g. anti-PD-1 or PD-L1), or a combination thereof.

The term "antigen-binding peptide", as used herein, relates to a peptide which specifically binds to an antigen. In one embodiment, an antigen-binding molecule is based on an immunoglobulin, such as a polyclonal or monoclonal antibody, or based on a protein scaffold structure having antigen-binding capacity, such as an anticalin protein, an Affilin, an Affimer, an Affitin, an Alphabody, a nanobody, or a DARPin. In one embodiment, an antigen-binding peptide relates to an antibody or an antibody fragment. In one embodiment, the terms "antibody", "antibody fragment", and "antigen-binding peptide" are used interchangeably. There are several classes of antibodies, particularly human antibodies, such as IgA, IgG, IgM, IgD and IgE, as well as subclasses, such as IgG1, IgG2, IgG3, IgG4, IgA1, IgA2, IgM, IgD, and IgE. Also useful for the invention may be IgGs that are hybrid compositions of the natural human IgG isotypes. In one embodiment, an antigen-binding peptide and/or antibody may comprise a domain of any antibody class, subclass, and/or chain. In one embodiment, the amino acid sequence of an antigen-binding peptide may be modified by protein engineering, e.g. to comprise constant regions from other immunoglobulin classes mediating improved binding properties, e.g. effector function properties, such as immune cell stimulating or immune cell inhibiting functions.

According to the present invention, an antigen-binding peptide may relate, for example, to human antibodies, human-chimeric antibodies, humanized antibodies, and antibody fragments. An antibody recognizes an antigen via the Fab fragment variable region comprising a paratope. Said paratope specifically targets an epitope on an antigen. According to the present invention, an antigen-binding peptide may further relate to a fragment of an antibody, such as a substantially intact antibody, a Fab fragment, a F(ab')₂ fragment, a diabody, a single chain Fv fragment, a tetrabody, a triabody, a disulfide bond-stabilized Fv (dsFv), or a heavy chain VHH fragment from camels. However, other forms of an antigen-binding peptide are also envisioned by the present invention. For example, the antigen-binding peptide may be another (non-antibody) receptor protein derived from small and robust non-immunoglobulin scaffolds, such as those equipped with binding functions for example by using methods of combinatorial protein design. Such a non-antibody antigen-binding peptide may be, for example, an Affibody molecule, an Affilin, an Affimer, an Affitin, an Alphabody, an Anticalin, a nanobody, or a DARPin. An antigen-binding peptide may also relate to other antibody-mimetic molecules such as a dual-affinity re-targeting antibody (DART). In one embodiment, an antigen-binding peptide preferably relates to an antibody or an antibody fragment. In one embodiment, an antigen-binding peptide may relate to any antibody-mimetic molecule which mimics the binding properties of an antibody to an antigen but which is structurally distinct from a naturally occurring antibody.

The term "human antibody", as used herein, relates to an antibody which only comprises human sequences, or a fragment thereof. In one embodiment, a human antibody is produced by a display technique, such as by phage display or ribosome display. The term "humanized antibody", as used herein, relates to an immunoglobulin chain or fragment thereof, such as a Fab fragment, a Fab' fragment, a F(ab')2 fragment, a Fv fragment, or another antigen-binding fragment of an antibody, which contain a sequence from non-human immunoglobulin. In one embodiment, a humanized antibody is a human antibody in which certain CDR residues are replaced by CDR residues from a non-human having desired specificity and affinity properties. The term "chimeric-human antibody", as used herein, relates to an artificial antibody which has been designed by genetic engineering or protein engineering by combining sequences of different species, such as mouse and human. In one embodiment, a human-chimeric antibody is a hybrid protein of the antigen-binding domain from a mouse antibody and the constant domain of a human antibody. The term "substantially intact antibody", as used herein, relates to an antibody which is not fragmented, truncated or otherwise shortened, and which has retained its capability of specifically binding to a target such as an antigen.

The term "targeting", as used herein, relates to the capacity of a molecular structure, such as an antibody, an antigen-binding peptide, or a surface molecule of a cytotoxic T cell, to bind to a certain structure, such as an antigen, specifically an epitope, by specific interaction. In one embodiment, a molecule on the surface of an immune cell is targeted by a first targeting agent, e.g. an antigen-binding peptide, and further molecules, e.g. at least two molecules, on the surface of a target cell, e.g. cancer cell, are targeted by a second and a third targeting agent. Such combined binding of a first targeting agent to a first molecule on an immune cell, and such binding of a second targeting agent to a second molecule on a target cell, e.g. cancer cell, and such binding of a third targeting agent to a third molecule on said target cell, results in the immune cell and the target cell being brought into proximity, thereby allowing for a formation of a immunological synapse. In one embodiment, such approximation of an immune cell and a target cell results in the activation of an effector system, which in turn results in the elimination of said target cell, e.g. cancer cell, for example via a caspase cascade. In one embodiment, the formation of an immunological synapse results in cell death of said cancer cell. In one embodiment, the terms "targeting" and "recognizing" are used interchangeably. The term "cross-reacting", as used herein, relates to off-target binding to a structure and/or molecule which is not the target. In one embodiment, the multifunctional nanostructure of the invention reduces cross-reactions, since the immune cell is only recruited in case at least two target molecules on said target cell are bound, and thus the specificity is increased, and off-target binding and side effects are reduced.

The term "first targeting agent", as used herein, relates to a molecule binding to and/or configured to bind to a first target, e.g. a first target cell. In one embodiment, a first target cell is an immune cell, or other effector cell. In one embodiment, said first targeting agent binds, preferably specifically binds, to a target molecule on an immune cell such as a lymphocyte e.g. T cell or natural killer cell, or a macrophage or neutrophil. In one embodiment, said first targeting agent is capable of activating the immune cell and/or is capable of stimulating an immune response of the immune cell, in which the immune response is preferably a cytotoxic immune response, for example, a cytotoxic immune response against a second target cell. In one embodiment, said first target molecule is preferably a molecule such as CD3, CD3δ/ε, CD3γ/ε, TCR, TCRα, TCRβ, CD2, CD5, CD28, OX40, 4-1BB, CD16, Ly49, NKp30 (CD337), NKp44 (CD336), NKp46 (NCR1), CD3ζ (CD247), CD27, CD40, CD137, CD64, CD89, toll-like receptors (TLR), cytokine receptors, a Fc domain, an engineered Fc domain, GITR, and ICOS. In one embodiment, the term "at least one" in the context of a targeting agent, e.g. a first targeting agent, means that one or more molecules of one type of said targeting agent are present; furthermore, additional targeting agents may be present, such as fourth, fifth or sixth targeting agent, wherein each of said fourth, fifth, or sixth targeting agent is one or more molecules of one type.

The terms "second targeting agent" and "third targeting agent", as used herein, relate to targeting agents binding to and/or configured to bind to a second target molecule and a third target molecule, respectively. In one embodiment, the second target molecule and the third target molecule are target molecules of the same target, e.g., the same target cell. In one embodiment, using a second targeting agent and a third targeting agent increases the specificity of a nanostructure comprising such as second and third targeting agents over a structure using only one targeting agent. In one embodiment, the specificity and selectivity of a nanostructure of the invention is increased compared to a nanostructure targeting only one target molecule on a target cell such as a second target cell. In one embodiment, a second target cell, such as a diseased cell e.g. cancer cell is only targeted, and optionally destroyed by cytolytic lysis, if both the second target molecule and the third target molecule are present on said second target cell. Accordingly, a nanostructure of the invention is advantageous in that it reduces off-binding by increasing the specificity and/or selectivity of cell targeting. Furthermore, a nanostructure the invention is advantageous in that it allows to specifically and/or selectively target and/or eliminate diseased cells. An advantage of the invention is that a nanostructure can specifically and/or selectivity trigger lysis of diseased cells, such as cancer cells and/or cells involved in an autoimmune response.

In one embodiment, a nanostructure comprises further targeting agents in addition to said first, second, and third targeting agent. In one embodiment, a structure comprises further targeting agents in addition to a first targeting agent, a second targeting agent, and a third targeting agent, e.g. a fourth targeting agent, and/or a fourth and fifth targeting agent, and/or even further targeting agents. In one embodiment, a nanostructure comprises at least a first targeting agent, a second targeting agent, and a third targeting agent, and further comprises further targeting agents.

In one embodiment, the term "immunological synapse" relates to an interface formed upon recognition between a first cell and a second cell, preferably mediated via a nanostructure of the invention, for example receptor-mediated and/or nanostructure-mediated recognition between an immune cell and an antigen presenting cell and/or cancer cell. In one embodiment, and immunological synapse is a cytolytic synapse which means that at least one cell is lysed upon formation of the immunological synapse. In one embodiment, a nanostructure of the invention induces synapse formation e.g. a formation of a synapse that is or is similar to a cytolytic synapse described in [11] or [12]. In one embodiment, an immunological synapse is a cytolytic T cell synapse, and/or a stimulatory synapse formed between a naive T cell as a first target and an a second target cell, e.g. an antigen-presenting cell, and/or a lytic synapse formed between a cytotoxic effector T cell as a first target and a second target cell.

In one embodiment, the term "synapse formation" relates to forming a synapse and/or a synaptic distance between a first target and a second target, e.g. between a first target cell and a second target cell. In one embodiment, such synapse formation is a formation of an immunological synapse. In one embodiment, such synapse formation, preferably immunological synapse formation, results in lysis and/or binding of the second target. In one embodiment, a nanostructure of the invention is used to induce and/or promote a synapse formation between two cells, e.g. a first target cell and a second target cell, for example between an immune cell and a cancer cell. In one embodiment, a nanostructure of the invention is used to bind a cell to a first target, such as a sensor. In one embodiment, a nanostructure of the invention is for use in inducing, promoting, stabilizing, and/or inhibiting synapse formation, preferably only if the first, second, and third targeting agents have bound to their respective target molecules. In a preferred embodiment, a nanostructure of the invention is for use in inducing and/or promoting synapse formation. In one embodiment, a nanostructure of the invention is for use in promoting a specific distance between two cells and/or between a cell and a target such as a surface, e.g. sensor surface. In one embodiment, a synapse is an immunological synapse. In one embodiment, a nanostructure is for use in inducing and/or promoting a formation of a synapse, preferably immunological synapse. In one embodiment, a nanostructure is used for conditional synapse formation, i.e. only if the condition of a first targeting agent binding to a first target molecule, a second targeting agent binding to a second target molecule, and a third targeting agent binding to a third target molecule, such synapse is formed. In an alternative embodiment, a nanostructure of the invention is for use in inhibiting synapse formation, e.g. for inhibiting an autoimmune response. In one embodiment, such inhibition of a synapse formation comprises inducing and/or stabilizing synapse formation and inhibiting a biological effect such synapse formation has on a first or second target, for example inhibiting that a first target cell attacks a second target cell, e.g. inhibiting that an immune cell attacks a cell, e.g. healthy cell or diseased cell, in an autoimmune response. In one embodiment, such inhibition of a biological effect can be implemented by said first targeting agent, in such case e.g. an immune-cell inhibiting agent, and/or by a further agent coupled to said first surface, preferably an immune-cell inhibiting agent coupled to said first surface. In one embodiment, such inhibition of a synapse formation is a specific inhibition of synapse formation, as said first or second target is inhibited only if said first targeting agent is bound to said first target molecule, said second targeting agent is bound to said second target molecule, and said third targeting agent is bound to said third target molecule.

In an alternative embodiment, a nanostructure of the invention inhibits synapse formation, e.g. nonspecifically inhibits synapse formation, 1) if a second target comprises neither a second nor a third target molecule, 2) if a second target comprises only a second target molecule but not a third target molecule, or 3) if a second target comprises only a third target molecule but not a second target molecule, for example by steric hindrance and/or an absence of binding specificity. In one embodiment, a nanostructure of the invention inhibits synapse formation, preferably by steric hindrance and/or binding specificity, between targets other than a first target comprising a first target molecule and a second target comprising a second target molecule and a third target molecule.

In one embodiment, the term "synaptic distance", as used herein, relates to a distance of a synapse, e.g., a distance of a synaptic cleft and/or a distance of a synaptic gap. In one embodiment, a synaptic distance is the distance between a first target and a second target when a synapse is formed. In one embodiment, a synaptic distance is a distance between two cells, wherein such distance is small enough to enable a first cell to have an effect on the second cell and/or to enable biochemical and/or electrochemical communication between a first cell and a second cell. In one embodiment, a synaptic distance is smaller than 25 nm, e.g. ≤ 20 nm, ≤ 15 nm, or ≤ 10 nm. In one embodiment, a synaptic distance is ≤ 25 nm, preferably ≤ 15 nm, more preferably ≤ 10 nm. In one embodiment, a synaptic distance is in the range of from 1 nm to 25 nm, preferably 5 nm to 15 nm, e.g. about 13 nm to 15 nm or 5 nm to 13 nm. In one embodiment, a natural synapse between a T cell and an antigen presenting cell has a synaptic distance in the range of from 13 to 15 nm. In one embodiment, artificial synapses, such as synapses induced by IgG, BiTe, or nanostructures have a synaptic distance in a range similar to a natural synapse, or smaller or larger, preferably similar or smaller, more preferably smaller, than a natural synaptic distance. In one embodiment, the smaller the synaptic distance, the better the effect, e.g. the cytolytic effect of an immune cell on a diseased cell. In one embodiment, said first target, e.g. first target cell, and said second target, e.g. second target cell, are bound using said nanostructure such that, as a result of said binding, said first target and said second target have a distance ≤ 25 nm, preferably ≤ 15 nm, more preferably ≤ 10 nm.

The invention relates nanostructures that can trigger the formation of synapses such as immune synapses between target cells and immune cells upon antigen-pattern recognition on the target cell. For example, immune cells capable of forming immunological synapses are T cells, B cells, and natural killer cells. In one embodiment, a synapse is formed between a cytotoxic T cell or natural killer cell and a target cell of interest, such as a cancer cell. In one embodiment, such synapses allow cytolytic molecules, e.g. cytolytic molecules released by a cytotoxic T cell or natural killer cell, to be targeted against the target cell, thereby causing target-cell death. In one embodiment, a nanostructure is used to form such cytolytic synapse in a method of preventing or treating cancer. In one embodiment, a nanostructure of the invention allows to kill tumor cells by inducing the formation of an immunological synapse between an immune cell and a tumor cell. In one embodiment, a nanostructure is also useful in applications, in which the immune system needs to be locally modulated; for example in auto-immune diseases. The nanostructure of the invention can be used in any application in which cells have to be brought in contact and/or in proximity to each other and/or applications in which cells have to be brought in contact and or in proximity to a target such as a sensor surface. In one embodiment, a nanostructure is used for forming electrical or electrochemical synapses between neurons and/or cells of the central nervous system. In one embodiment, a nanostructure is used for forming electrical or electrochemical synapses between neurons and/or cells of the central nervous system and electrical and/or electrochemical stimulating means, such as electrical and/or electrochemical stimulating devices.

In one embodiment, a nanostructure is for use in inducing, promoting, stabilizing, and/or inhibiting a formation of a synapse, preferably only if the first, second, and third targeting agents have bound to their respective target molecules. In one embodiment, a synapse is formed only if the first, second, and third targeting agents have bound to their respective target molecules. In one embodiment, a first target cell and a second target cell are bound using said nanostructure only if the first, second, and third targeting agents have bound to their respective target molecules. In one embodiment, a method of preventing or treating a disease comprises binding a first target cell and a second target cell using said nanostructure only if the first, second, and third targeting agents have bound to their respective target molecules. In one embodiment, a method of preventing or treating a disease comprises inducing, promoting, stabilizing, and/or inhibiting a formation of a synapse only if the first, second, and third targeting agents have bound to their respective target molecules. In one embodiment, a use of a nanostructure comprises inducing, promoting, stabilizing, and/or inhibiting a formation of a synapse between a first target cell and a second target cell only if the first, second, and third targeting agents have bound to their respective target molecules. In one embodiment, a use of a nanostructure comprises binding said first target, e.g. first target cell, and said second target, e.g. second target cell, using said nanostructure such that, as a result of said binding, said first target and said second target have a distance ≤ 25 nm, preferably ≤ 15 nm, preferably only if the first, second, and third targeting agents have bound to their respective target molecules. In one embodiment, the "respective target molecule" of a first targeting agent is a first target molecule, the "respective target molecule" of a second targeting agent is a second target molecule, and the "respective target molecule" of a third targeting agent is a third target molecule. In one embodiment, a formation of a synapse is induced, promoted, stabilized, and/or inhibited only if the second target, preferably second target cell, presents at least one second target molecule and at least one third target molecule. In one embodiment, a use of a nanostructure comprises inducing, promoting, stabilizing, and/or inhibiting a formation of a synapse between a first target cell and a second target cell, preferably only if the second target, preferably second target cell, presents at least a second target molecule and at least a third target molecule. In one embodiment, a method of preventing or treating a disease comprises inducing, promoting, stabilizing, and/or inhibiting a formation of a synapse only if the second target, preferably second target cell, presents at least one second target molecule and at least one third target molecule. In one embodiment, a nanostructure is a conditional cell connector, i.e. conditional nanostructure, as it induces, promotes, stabilizes, and/or inhibits a formation of a synapse only if the first, second, and third targeting agents have bound to their respective target molecules. In one embodiment, a "condition" for a binding of a first target and a second target is that the first, second, and third targeting agents have bound to their respective target molecules. In one embodiment, a "condition" for a binding of a first target is that the second and third targeting agents have bound to their respective target molecules.

The term "nanostructure", as used herein, relates to a nucleic acid nanostructure, preferably a DNA-origami structure which is composed of one or multiple DNA-origami subunits (entities). In embodiment, the nanostructure is a DNA origami nanostructure. Readily available nucleic acid nanostructure techniques e.g. DNA origami techniques, which involve comparatively less complex procedures to assemble than standard nanomanufacturing techniques, may be used to manufacture the nanostructure. In one embodiment, the nanostructure is at least partially manufactured using DNA origami techniques. Owing to the self-assembly of DNA origami structures and the readily available software for designing the corresponding scaffolding and staple strands, this may be a comparatively less complex manufacturing process compared to standard nanomanufacturing techniques. In one embodiment, a nanostructure is a DNA origami structure. In one embodiment, a nanostructure comprising targeting agents is a nanostructure with said targeting agents attached thereto. For example, said targeting agents can be attached to said nanostructure via any surface of said nanostructure and/or via a core structure of said nanostructure, e.g. via an inner part of said core structure and/or nanostructure. In one embodiment, a nanostructure comprising targeting agents is a DNA origami with said targeting agents attached thereto. In one embodiment, a core structure of a nanostructure comprising targeting agents is a DNA origami, and to such core structure being a DNA origami said targeting agents are attached. In one embodiment, the targeting agents are not part of the core structure, but are attached to the core structure e.g. a DNA origami, optionally via a nucleic acid strand conjugated to the targeting agents. In one embodiment, a nanostructure has a maximum length smaller than 1000 nm, e.g. in the range of from about 25 to about 70 nm, preferably about 30 nm to about 50 nm. In one embodiment, a maximum length is along a longitudinal axis. In one embodiment, a maximum length is larger than a synaptic distance, e.g. ≥ 25 nm.

In one embodiment, a DNA-origami structure comprises at least one scaffolding strand and a plurality of single-stranded oligonucleotide staple strands. In one embodiment, a scaffolding strand makes up and/or traverses the main part of a DNA-origami structure and/or a DNA-origami subunit ("entity"). In one embodiment, an entity is a DNA-origami subunit. The term "staple strand", as used in this invention, shall refer to a single-stranded oligonucleotide molecule, which is at least partially complementary to a scaffolding strand. In general, staple strands can be used to introduce, e.g., coupling sites into a DNA-origami structure and/or a DNA-origami subunit ("entity").

This invention generally relates to nanostructures. In examples of the present invention, the nanostructure may be a DNA origami structure, i.e., a DNA origami device, as exemplarily disclosed in US 7,842,793 B2. However, it should be understood that the present technology is not limited thereto, and it should be understood that this is merely exemplary and that also other nanostructures may be utilized to exercise the present invention. However, as used herein, the term "nanostructure" shall preferably refer to a DNA-origami structure, which is composed of one or more DNA-origami subunits (entities).

The nanostructure, e.g. DNA origami nanostructure, may comprise at least one scaffolding strand, i.e. single-stranded polynucleotide scaffold DNA with a known sequence. The DNA origami structure may further comprise a plurality of single-stranded oligonucleotide staple strands, wherein each staple strand may be at least partially complementary to at least one scaffolding strand. Further, each of the staple strands may be configured to bind to the at least one scaffolding strand, wherein the at least one scaffolding strand may be folded and/or arranged such that the desired nanostructure may be formed. The term "strand", as used herein, relate to a nucleic acid strand, e.g. a DNA and/or RNA strand. Such a three-dimensional nanostructure may be realized using DNA origami, i.e. by combining scaffolding strands and staple stands to form the required portions and the overall device. Such designs may for example be performed using software such as caDNAno. That is, a nanostructure comprising multiple portions may in some embodiments be made out of one scaffolding strand, whereas in other embodiments portions of a nanostructure may be constructed utilizing a plurality of scaffolding strands.

In embodiments where the nanostructure is at least partially formed by a DNA origami structure and wherein the DNA origami structure comprises at least one scaffolding strand and a plurality of staple strands, the molecule may be bound to one of the at least one scaffolding strand or a staple strand by means of a linker molecule, wherein the linker molecule may be connected to a DNA strand portion, which is complementary to a portion of the at least one scaffolding strand or to a portion of a staple strand. In one embodiment, the first targeting agent is coupled to the nanostructure via a linker molecule, and/or the second targeting agent is coupled to the nanostructure via a linker molecule, and/or the third targeting agent is coupled to the nanostructure via a linker molecule. In one embodiment, a linker molecule is one or more nucleic acid bases, e.g. a single nucleic acid base or a nucleic acid sequence. In one embodiment, a linker molecule of a targeting agent is connected to a DNA strand of said nanostructure.

In one embodiment, the shape of a nanostructure may be any shape, for example, a rod, a triangular shape, a round shape, a cuboid, i.e. a rectangular shape, a star shape, or any other shape. In one embodiment, the nanostructure is not a globular structure. The nanostructure of this invention can be of any length. In a preferred embodiment, the nanostructure comprises a maximum length, and in a particularly preferred embodiment, the maximum length is smaller than 1000 nm, preferably smaller than 500 nm, such as around 100 nm, or smaller.

In one embodiment, the nanostructure comprises at least one nucleic acid, wherein said nucleic acid may comprise at least one modification, for example a chemical modification selected from a modified internucleoside linkage, a modified nucleobase, or a modified sugar moiety, such as a 2'-O-alkyl modification, for example a 2'-O-methoxy-ethyl (MOE) or 2'-O-Methyl (OMe) modification, an ethylene-bridged nucleic acid (ENA), a 2'-fluoro (2'-F) nucleic acid, such as 2'-fluoro N3-P5'-phosphoramidite, a 1',5'-anhydrohexitol nucleic acid (HNA), or a locked nucleic acid (LNA).

The term "rigid", as used herein, relates to a feature of a rigid body, e.g. a rigid nanostructure, which means that such body, e.g. nanostructure, is a solid body in which deformation is zero or so small that it can be neglected. The distance between any two given points on a rigid body, e.g. nanostructure, remains constant in time regardless of external forces exerted on it. A rigid body is usually considered as a continuous distribution of mass. In one embodiment, when referring to a nucleic acid being "rigid", such term "rigid" relates to the nanostructure having only one configuration, i.e. not being a flexible nanostructure having more than one configuration, e.g. flexible via a hinge region. In one embodiment, such "rigidity" and/or "flexibility" and/or "only one configuration" relates to a core structure of the nanostructure, e.g. a DNA origami, i.e. the nanostructure itself, but does not relate to the comprised, attached, and/or bound targeting agents. For example, a linkage between a nanostructure, such as DNA origami, and a targeting agent may be flexible and/or may comprise a hinge region, while the nanostructure itself, e.g. the DNA origami, is rigid, i.e. non-flexible. In one embodiment, when referring to a nanostructure having only one configuration, such configuration being only one configuration, relates to the nanostructure itself, e.g. a core structure of the nanostructure, such as a DNA origami, but does not relate to the targeting agents and/or the linkage between the targeting agents and the nanostructure. For example, a linkage between a targeting agent and a nanostructure, and/or a targeting agent, may have more than one configuration. In one embodiment, such linkage between a targeting agent and a nanostructure is via a linker and/or via a nucleic acid attached to said targeting agent. In one embodiment, the nucleic acid nanostructure of the invention does not have an open and closed configuration. In one embodiment, the nucleic acid nanostructure of the invention has only one configuration. In one embodiment, said nanostructure does not have a hinge region. In one embodiment, said nanostructure does not have more than one conformational state. In one embodiment, the terms "nanostructure" and "nucleic acid nanostructure" are used interchangeably. In one embodiment, when referring to a "core structure" of a nanostructure, the nucleic acid nanostructure without the targeting agents is meant, e.g. a DNA origami comprised by a nucleic acid nanostructure of the invention. In one embodiment, when referring to a nanostructure "without a hinge region", a nanostructure which does not have a hinge region in a core structure of the nanostructure is meant; i.e. the nucleic acid nanostructure does not comprise a hinge region in the nucleic acid core structure, but may have a hinge region between the targeting agents and the core structure. In one embodiment, when referring to a nanostructure having only one configuration, this implies that such nanostructure, preferably a core structure of such nanostructure e.g. a DNA origami comprised by the nanostructure, cannot switch between states. In one embodiment, a nanostructure has the same configuration and/or same state when bound to a first and/or second target and when not bound to a first and/or second target. In one embodiment, a nanostructure does not have an "open" and "closed" configuration. In one embodiment, the accessibility of a targeting agent does not depend on a configuration of the nanostructure, but depends on the location, alignment, and/or orientation of the nanostructure with regard to a first target and a second target.

In one embodiment, each of the staple strands is configured to bind to at least one of the at least one scaffolding strand in at least one, preferably two or more distinct places. In one embodiment, a nucleic acid nanostructure and/or a DNA origami comprised by a nucleic acid nanostructure has/have a scaffolding strand and one or more staple strands. In one embodiment, a nucleic acid nanostructure and/or a DNA origami comprised by a nucleic acid nanostructure comprise(s) ≤ 100 staple strands. In one embodiment, staple strands have a length of from 50 to 150 nucleic acid bases. In one embodiment, a scaffolding strand has a length of from 100 to 20000 nucleic acid bases, preferably from 120 to 10000 nucleic acid bases, more preferably from 150 to 8064 nucleic acid bases. In one embodiment, a targeting agent is attached and/or bound to the nucleic acid nanostructure via a nucleic acid sequence conjugated to said targeting agent, preferably a nucleic acid sequence that is complementary to a nucleic acid sequence of the nucleic acid nanostructure, e.g. complementary to a scaffolding strand of said nucleic acid nanostructure, and/or is complementary to a nucleic acid handle, preferably DNA handle, attached to and/or incorporated in said nucleic acid nanostructure.

At least three interaction sites, namely at least three targeting agents, specifically a first targeting agent, a second targeting agent, and a third targeting agent, are attached to the nanostructure. The targeting agents are attached in such a way, and the nanostructure is designed in such a way, that when a subset A of the targeting agents, preferably at least one of the second and the third targeting agent, is not bound their respective targets, the nanostructure is in an orientation in which a subset B of the targeting agents, preferably the first targeting agent, cannot bind to their respective targets due to steric hindrance. Once the subset A of the interaction molecules, preferably the second targeting agent and the third targeting agent, binds to their respective targets, the nanostructure becomes oriented in an accessible orientation in which the subset B of targeting agents, preferably the first targeting agent, can bind their respective targets. Therefore, the binding rate of the subset B of targeting agents on the nanostructure is conditionally modified by the binding state of the subset A of targeting agents. Accordingly, the conditional orientation is mediated through the binding of the targeting agents.

The terms "first surface" and "second surface", as used herein, relate to opposing surfaces of a nanostructure, particularly opposing surfaces along a longitudinal axis of the nanostructure. In one embodiment, a "surface", e.g. first surface and/or second surface, relates to a surface which can be, for example, in the form of an area, line, edge, corner, DNA helix blunt-end, DNA and/or a point. In one embodiment, a surface has any topology. In one embodiment, a surface is a two-dimensional or three-dimensional surface. In one embodiment, a surface e.g. first surface or second surface is an interrupted surface or non-interrupted surface. In one embodiment, an interrupted surface has holes, bumps, recesses, and/or indentations within said surface, e.g. holes and/or indentations formed by a recess. In one embodiment, a surface, e.g. first surface or second surface, is an area which comprises at least one recess, wherein, preferably, said targeting agent(s) is/are comprised within such recess, wherein, preferably, at least a portion of said targeting agent(s) is/are presented on said surface.

In one embodiment, said nanostructure comprises a third surface and a fourth surface, wherein said third and fourth surface are opposing surfaces along a transverse axis of the nanostructure, and/or wherein said third and fourth surface are abutting surfaces to said first and/or second surface, and/or wherein said third and fourth surface are perpendicular to at least one of said first and said second surface. In one embodiment, said third surface is separated from at least one of said first and second surfaces via an edge. In one embodiment, said fourth surface is separated from at least one of said first and second surfaces via an edge. In one embodiment, a first targeting agent is attached to a third surface or fourth surface and/or to an edge, wherein said edge separates a third surface from a first or second surface or a fourth surface from a first or second surface. In one embodiment, a second and/or third targeting agent is/are attached to a third surface or fourth surface and/or to an edge, wherein said edge separates a third surface from a first or second surface or a fourth surface from a first or second surface. In one embodiment, if a second or third targeting agent is attached to said third surface or fourth surface and/or to said edge, at least a portion of said second or third targeting agent is presented on said second surface. In one embodiment, if a first targeting agent is attached to said third surface or fourth surface and/or to said edge, at least a portion of said first targeting agent is presented on said first surface.

The terms a "surface comprises" and "surface comprises a targeting agent", in the context of a surface such as a first or second surface comprising a targeting agent, e.g. a first targeting agent, and a second and third targeting agent, respectively, means that said surface presents at least a portion of said targeting agent, preferably presents at least a portion of said targeting agent on its surface, and/or that at least a portion of said targeting agent is accessible from said surface. In one embodiment, the term "accessible", as used herein, means capable of binding to and/or being bound by a target molecule. In one embodiment, the term a targeting agent being "accessible from", in the context of a surface, means that such surface does not sterically hinder the accessibility of such targeting agent.

In one embodiment, the term a "first surface comprises at least a first targeting agent" means that at least a portion of said at least a first targeting agent is presented on said first surface and/or that at least a portion of said first targeting agent is accessible from said first surface. In one embodiment, said first targeting agent is attached to said nanostructure on a surface, such as on said first surface, and/or below a surface, such as below said first surface, e.g. attached within a core structure of said nanostructure. In one embodiment, said first targeting agent is attached to said nanostructure on a surface or below a surface, such as the first surface, e.g. within a recess or below a surface of said recess. In one embodiment, a first targeting agent can be attached to any part of the nanostructure, e.g. attached within a core of said nanostructure and/or attached to a plane and/or surface perpendicular to the first or second surface, e.g. attached to a third or fourth surface, to an attachment plane, or to an attachment site. In one embodiment, a first targeting agent can be attached to any part of the nanostructure provided that at least a portion of said first targeting agent is presented on a first surface.

In one embodiment, the term a "second surface comprises at least a second targeting agent and at least a third targeting agent" means that at least a portion of said at least a second targeting agent and at least a portion of said at least a third targeting agent are presented on said second surface, and/or that at least a portion of each of said second and third targeting agents is accessible from said second surface. In one embodiment, said second targeting agent and/or third targeting agent is/are attached to said nanostructure on a surface, such as on a second surface, and/or below a surface, such as below said second surface, e.g. attached within a core structure of said nanostructure, e.g. in an inner part of said nanostructure. In one embodiment, said second targeting agent and/or third targeting agent is/are attached to said nanostructure on a surface or below a surface, such as said second surface, e.g. within a recess or below a surface of said recess. In one embodiment, the second targeting agent and/or third targeting agent can be attached to any part of the nanostructure, e.g. attached within a core of said nanostructure and/or attached to a plane and/or surface perpendicular to the first or second surface. In one embodiment, the second targeting agent and/or third targeting agent can be attached to any part of the nanostructure provided that at least a portion of said second targeting agent and at least a portion of said third targeting agent is/are presented on a second surface.

In one embodiment, the term "presented on" refers to "exposed on", e.g. at least a portion of a targeting agent is presented on a surface and/or exposed on said surface. In one embodiment, a portion of a targeting agent presented on and/or exposed on a surface is capable of binding to its respective target molecule. In one embodiment, a portion of a targeting agent presented on and/or exposed on a surface is accessible for the respective target molecule. In one embodiment, the nanostructure of the invention is a nucleic acid nanostructure comprising a first surface and a second surface, wherein said first surface and said second surface are located at opposing sides of said nanostructure, wherein said first surface comprises at least a first targeting agent, i.e. said first surface presents at least a portion of at least a first targeting agent, and said second surface comprises at least a second targeting agent and at least a third targeting agent, i.e. said second surface presents at least a portion of at least a second targeting agent and at least a portion of at least a third targeting agent.

In one embodiment, the first targeting agent is attached to the nanostructure within a plane that is parallel to and/or close to (e.g. 0-5 nm) the first surface of the nanostructure. In one embodiment, the first targeting agent is attached to the nanostructure at an attachment site and/or attachment plane which is parallel to and/or close to (e.g. 0-5 nm) the first surface of the nanostructure. In one embodiment, such attachment site and/or attachment plane intersects with a third or fourth surface, wherein, optionally, said first targeting agent is attached to the nanostructure at such intersection.
In one embodiment, the second and/or third targeting agent is/are attached to the nanostructure within a plane that is parallel to and/or close to (e.g. 0-5 nm) the second surface of the nanostructure. In one embodiment, the second and/or third targeting agent is/are attached to the nanostructure at an attachment site and/or attachment plane which is parallel to and/or close to (e.g. 0-5 nm) the second surface of the nanostructure. In one embodiment, such attachment site and/or attachment plane intersects with a third or fourth surface, wherein, optionally, said second and/or third targeting agent is/are attached to the nanostructure at such intersection.

In one embodiment, a first targeting agent is attached to a plane and/or site and/or surface which is not an outermost surface, e.g. not an outermost first surface. In one embodiment, a second and/or third targeting agent is/are attached to a plane and/or site and/or surface which is not an outermost surface, e.g. not an outermost second surface. In one embodiment, the first and/or second surface is/are not the outermost surfaces of the nanostructure along the longitudinal axis, e.g. may be a surface of a recess. In an alternative embodiment, the first and/or second surface is/are the outermost surfaces of the nanostructure along the longitudinal axis. In a preferred embodiment, at least a portion of the first targeting agent is presented on and/or accessible on an outermost surface of said nanostructure, e.g. a first surface. In a preferred embodiment, at least a portion of the second and/or third targeting agent(s) is/are presented on and/or accessible on an outermost surface of said nanostructure, e.g. a second surface.

In one embodiment, the first targeting agent is attached to the nanostructure, e.g. DNA-origami object, either directly on the first surface, and/or is attached to the nanostructure at an edge of said first surface, e.g. an edge separating said first surface from a third or fourth surface, and/or is attached to the nanostructure at a corner of said first surface and an adjacent surface, e.g. a third of fourth surface. In one embodiment, the second and/or third targeting agent is/are attached to the nanostructure, e.g. DNA-origami object, either directly on the second surface, and/or is/are attached to the nanostructure at an edge of said second surface, e.g. an edge separating said second surface from a third or fourth surface, and/or is/are attached to the nanostructure at a corner of said second surface and an adjacent surface, e.g. a third of fourth surface. In one embodiment, the terms "adjacent surface" and "abutting surface" are used interchangeably. In one embodiment, said first targeting agent is attached to said nanostructure at a location having a distance, preferably a distance along a transverse axis, of about 0-5 nm from said first surface and/or from a first outermost surface. In one embodiment, said second or third targeting agent is/are attached to said nanostructure at a location having a distance, preferably distance along a transverse axis, of about 0-5 nm from said second surface and/or from a second outermost surface. The first, second, and/or third targeting agents may also be placed and/or attached in attachment planes that are parallel to the first or second surface to obtain a smaller cell distance. The optimal distance of the attachment plane from the first or second surface may depend on the size of the targeting agent and on the distance between the target molecule's epitope from the cell surface. The distance of the parallel attachment plane to its corresponding surface may be chosen from 0-10 nm, preferably smaller than 5 nm, such as 2 nm.

The term "longitudinal axis", as used herein, relates to a line passing through the centroid of the transverse sections, and/or is a line passing through a maximum extension and/or a maximum length of a nanostructure, e.g. a maximum extension and/or a maximum length of a core structure of the nanostructure, and/or a principle axis of inertia. In one embodiment, when referring to said first surface and said second surface being located at "opposing sides along said longitudinal axis", such term relates to opposing sides along and parallel to said longitudinal axis. In one embodiment, a first surface and a second surface are in planes parallel to the longitudinal axis.

The term "transverse axis", as used herein, relates to an axis that is transverse to a longitudinal axis, e.g. perpendicular to a longitudinal axis and/or is a crosswise axis to a longitudinal axis. In one embodiment, a transverse axis and a longitudinal axis are oriented to each other as shown in Figures 1-4.

The term "maximum longitudinal extension", as used herein, relates to a maximum length of the nanostructure, preferably along the longitudinal axis. The term "maximum transverse extension", as used herein, relates to a length of the nanostructure in a transverse direction and/or crosswise direction, preferably a maximum length of the nanostructure in a transverse direction and/or crosswise direction. In one embodiment, a maximum length of the nanostructure along a transverse axis is smaller than the maximum length of the nanostructure which is the maximum length along a longitudinal axis. In one embodiment, the maximum transverse extension is smaller than the maximum longitudinal extension. In one embodiment, a maximum length along a transverse extension is smaller than a maximum length along a longitudinal extension.

The term "target molecule", as used herein, relates to any molecule of a target, such as a chemical compound, a peptide, and antigen, lipid, and/or any other molecule to be bound by a targeting agent. In one embodiment, the target molecule is a molecule presented on the surface of a target and/or accessible on the surface of the target. In one embodiment, a target molecule such as an antigen is a cell-surface bound molecule or a sensor-surface bound molecule, such as any of proteins, cluster-of-differentiation molecules, receptors, lipids, ion channels, sugars, or any other cell-surface bound molecule or structure. In one embodiment, a target molecule is an antigen.

The term "first target molecule", as used herein, relates to a target molecule on a first target, such as a target molecule presented on the surface of a first target, such as an immune cell, effector cell, sensor surface, or immobilization surface.

The term "first target", as used herein, relates to any molecule, structure, material, cell, antigen, and/or surface to be bound by a first targeting agent. In one embodiment, said first target is a cell, and/or a molecule, material, or surface e.g. a sensor surface, immobilization surface or other surface used for an assay. For example, a first target can be a sensor, an immobilization surface, a bead, a column material of a chromatography column, a microtiter plate surface, a biotinylated or streptavidin-coated surface, and/or any other surface, material, or molecule used for an assay to immobilize analytes such as cells. For example, a nanostructure can be targeted to a first target to immobilize the nanostructure and the second target binding thereto. Such immobilization can be used for performing assays, selecting and/or sorting cells, or any other method or assay known to a person skilled in the art. In a preferred embodiment, a first target is a cell, preferably an immune cell. In an alternative embodiment, the first target is a sensor surface or any other material used for an assay, e.g. for measuring on/off binding rates.

The term "first target cell", as used herein, relates to a cell, e.g. a cell to be brought into proximity with a second target cell. In one embodiment, the first target cell is an immune cell, such as a lymphocyte, e.g. T cell, cytotoxic T cell, B cell, natural killer cell, natural killer T cell, CAR-T cell, monocyte, macrophage, or neutrophil, or other effector cell. The term "effector cell", as used herein, refers to a cell, preferably an immune cell, which is involved in an effector system and/or effector mechanism resulting in the destruction and/or elimination of a target cell. The term "cytotoxic T cell", as used herein, relates to a T lymphocyte which is able to kill a target cell, such as a cancer cell, an infected cell or a cell which is damaged and/or pathological in any other way. A T cell typically expresses a T cell receptor (TCR) that specifically recognizes an antigen. Such an antigen is typically presented on the surface of a cell by an MHC-I molecule. In one embodiment, such antigen is the first targeting agent of the nanostructure. If the T cell receptor of a cytotoxic T cell is specific for an antigen, it binds to the complex of the antigen and the MHC-I molecule and destroys the cell. The interaction of a T cell with the complex of an antigen and a MHC-I molecule of a target cell may also involve a co-receptor, namely CD8. In one embodiment, the nanostructure comprises a fourth targeting agent, preferably on said first surface, which is or binds to CD8, preferably binds to CD8, more preferably CD8 on said first target cell. In one embodiment, a fourth a targeting agent is configured to bind to a fourth target molecule which is CD8. In one embodiment, when a T cell binds to such a complex on a target cell or if an immunological synapse is formed using a nanostructure of the invention, the T cell releases any of the cytotoxins perforin, granzyme, and granulysin. In one embodiment, through the action of perforin, granzymes enter the cytoplasm of the second target cell, e.g. diseased cell, and their serine protease function triggers a caspase cascade leading to apoptosis of said second target cell. In one embodiment, apoptosis of said second target cells may also be induced by cell-surface interaction between the first target cells e.g. cytotoxic T cell and the second target cell, such as by a Fas-Fas ligand interaction, and/or by formation of an immunological synapse.
The term "second target molecule", as used herein, relates to a target molecule on a second target, such as a target molecule presented on the surface of a second target, such as a diseased cell e.g. a cancer cell or cell involved in an autoimmune response.

The term "second target", as used herein, relates to any molecule, structure, material, cell, antigen, and/or surface to be bound by a second and/or third targeting agent, e.g. a target cell such as a diseased cell. In one embodiment, a second and a third targeting agent can be used for cell sorting by targeting said second target and said first target, preferably by targeting said second target molecule and third target molecule on said second target. In one embodiment, by having an immobilized first target, target cells, specifically target cells having a second target molecule and a third target molecule on their surface, can be selectively and/or specifically immobilized via said nanostructure. Furthermore, such second target, such as a diseased cell, can be eliminated and/or destroyed by targeting an immune cell, and/or effector cell against said second target via a nanostructure.

The term "second target cell", as used herein, relates to a target cell such as a diseased cell or a cell to be immobilized. The term "diseased cell", as used herein, relates to a cell having at least one pathological feature such as abnormal division or abnormal function, e.g. such cell is involved in a disease and/or causes a disease, such as cancer or an autoimmune disease. For example, if a diseased cell is a cancer cell, such cancer cells are cells that divide relentlessly, thereby forming tumors. For example, if a diseased cell is a cell involved in an autoimmune response, such cell can be a cell which evokes an autoimmune response and/or which is targeted by an autoimmune response. In one embodiment, a diseased cell is any cell involved in a disease and/or in the development of a disease, preferably a cell causing such disease and/or development of disease.

The term "third target molecule", as used herein, relates to a target molecule on a second target, such as a target molecule presented on the surface of a second target, such as a diseased cell e.g. a cancer cell or cell involved in an autoimmune response. In one embodiment, the second target molecule and the third target molecules are the same or different target molecules, preferably different target molecules. In one embodiment, by targeting at least two target molecules on a second target, namely a second target molecule and a third target molecule, a specificity and/or selectivity of targeting said second target is increased.

The term "spaced at a distance from each other", as used herein, relates to a spacing between at least two molecules and/or at least to two molecules being located at a distance from each other, e.g. a spacing of a second targeting agent and a third targeting agent along a longitudinal axis.

The term "surface molecule", as used herein, relates to a molecule which is presented on a surface and/or of which at least a portion is presented on a surface. The term "not identical", as used herein in the context of molecules, targets and/or agents, relates to molecules or entities not being the same, e.g. a second target molecule and a third target molecule not being the same molecule and/or the same type of molecule. The term "combination of target molecules", as used herein, relates to relates to a combination of a second target molecule and a third target molecule, preferably being a pair of surface molecules, target molecules, and/or biomarkers of a target cell. For example, such combination can be a combination of two biomarkers typically expressed on a particular diseased cell, such as a particular cancer cell.

The term "DNA origami structure", as used herein, relates to a nanostructure that comprises DNA as a building material to make nanoscale shapes. Preparing a DNA origami involves folding of one or more "scaffolding" DNA strands into a particular shape using a plurality of rationally designed "staple" DNA strands, e.g. by self-assembly. A scaffolding strand is typically longer than a staple strand. The nucleic acid sequences of the staple strands are designed such that the staple strands hybridize to particular portions of the scaffolding strands and, due to the hybridization, a particular shape of the nanostructure is obtained. In one embodiment, the term nucleic acid "handle" relates to a nucleic acid sequence extension capable of binding to a moiety, e.g. a staple strand having extensions capable of binding to a moiety such as a nucleic acid sequence attached to a targeting agent.

The term "oriented perpendicular to a maximum longitudinal extension", as used herein, relates to being oriented in a transverse plane and/or perpendicular plane to the longitudinal extension and/or the longitudinal axis. In one embodiment, a recess may have any shape and/or form, such as a regular shape or an irregular shape.

The term "marker", as used herein, relates to any marker known to person skilled in the art, such as a marker and/or dye for in vitro assays or for in vivo medical imaging, e.g., a fluorescent marker, a radiography marker, an MRI marker, a nuclear medicine marker, a tomography marker.

The term "specific" or "specifically binding", as used herein, means in accordance with this invention that the targeting agent, e.g. antibody or antigen-binding peptide, is capable of specifically interacting with and/or binding to a specific target or a set of specific targets but does not essentially bind to other molecules, such as antigens. Such binding may be exemplified by the specificity of a lock-and-key-principle. In one embodiment, when referring to the term "binding", such term is to be construed to comprise the term "specific binding". In one embodiment, when referring to "targeting", such term is to be construed to comprise the term "specific targeting".

The term "nucleic acid", as used herein, relates to a nucleotide sequence, such as ribonucleic acid or deoxyribonucleic acid. The term "patient", as used herein, may relate to a human or an animal.

In one embodiment, the term "recess", as used herein, relates to a recess in a nanostructure. In one embodiment, a recess has a depth of from 0.25 nm to 9 nm, preferably 2 nm to 7 nm, more preferably about 5 nm. In one embodiment, a depth of such recess is oriented along the transverse axis of the nanostructure. In one embodiment, said first targeting agent is bound to said nanostructure via a recess and/or at least a portion of said first targeting agent is located within a recess; and/or said second targeting agent is bound to said nanostructure via a recess and/or at least a portion of said second targeting agent is located within a recess; and/or said third targeting agent is bound to said nanostructure via a recess and/or at least a portion of said third targeting agent is located within a recess. In one embodiment, the first targeting agent, the second targeting agent, and the third targeting agent are bound to the nanostructure via a recess and/or at least a portion of each of the first targeting agent, the second targeting agent, and the third targeting agent is located within a recess. In one embodiment, a nanostructure has one or more recesses. In one embodiment, incorporating a recess in a nanostructure even enhances the specificity of the nanostructure. In one embodiment, the second targeting agent and the third targeting agent are each incorporated into a recess. In one embodiment, if the second targeting agent and the third targeting agent are each incorporated into a recess, the first targeting agent first binds to its target molecule due to better accessibility of the first targeting agent, and subsequently the second and third targeting agents bind to their target molecules. In one embodiment, when referring to a targeting agent being "incorporated" into a recess, such "incorporation" comprises being bound to the nanostructure via said recess and/or at least a portion of said targeting agent being located within said recess. In one embodiment, the nanostructure comprises a recess that comprises said first targeting agent, said second targeting agent, or said third targeting agent. In one embodiment, the recess is oriented perpendicular to a longitudinal axis, which means it is oriented along a transverse axis.

In one embodiment, when referring to a method, the method is an in vivo, ex vivo, in vitro, or in situ method, e.g. an in vitro method. In one embodiment, when referring to a use, the use is an in vivo, ex vivo, in vitro, or in situ use, e.g. an in vitro use. In one embodiment, the invention relates to the use of a nanostructure for binding a first target and a second target, preferably for inducing, promoting, stabilizing, and/or inhibiting a formation of a synapse between said first target and said second target, more preferably for inducting and/or promoting a formation of a synapse between said first target and said second target, wherein said use is an in vivo, ex vivo, in vitro, or in situ use, e.g. an in vitro use. In one embodiment, the nanostructure of the invention is for use in vivo or in vitro, preferably in vivo.

Another aspect of this invention, which can be combined with any of the other preferred embodiments and/or aspects of the invention, pertains to a vector, in particular an expression vector, comprising a sequence coding for a nanostructure, a sequence of a scaffolding strand and/or a staple strand, and/or comprising the sequence of the nanostructure, as defined above.

The composition, preferably pharmaceutical composition, of the invention shall be formulated to be compatible with its intended route of administration. In a particularly preferred embodiment, examples of routes of administration of the pharmaceutical and/or compound of this invention include intravenous, oral, intranasal, intrathecal, intra-arterial, intradermal, subcutaneous, transdermal (topical), intracerebroventricular, intraparenchymal, intratumoral, transmucosal, rectal, vaginal, bronchial, parenteral administration, and any other clinically/medically accepted method for administration of a pharmaceutical and/or a compound.

Yet another aspect of this invention, which can be combined with any of the other aspects or specific embodiments of this invention, relates to a method of preventing and/or treating a disease, preferably a disease such as a proliferative disease, such as cancer, an immunological disorder, e.g. an autoimmune disease, a viral disease, such as a human immunodeficiency virus (HIV) infection, a metabolic disorder, an infectious disorder, and/or diabetes, the method comprising administering to a subject a therapeutically effective amount of a nanostructure, and/or a composition according to this invention.

The term "cancer", as used herein, refers to a disease characterized by dysregulated cell proliferation and/or growth. The term comprises benign and malignant cancerous diseases, such as tumors, and may refer to an invasive or non-invasive cancer. The term comprises all types of cancers, including carcinomas, sarcomas, lymphomas, germ cell tumors, and blastomas. The term "cancer cell", as used herein, refers to a cell that exhibits abnormal proliferation and divides relentlessly, thereby forming a solid tumor or a non-solid tumor.

Examples of autoimmune diseases include celiac disease, post-infectious IBS, diabetes mellitus type 1, Henloch Scholein Pupura (HSP) sarcoidosis, systemic lupus erythematosus (SLE), Sjogren syndrome, eosinophilic granulomatosis with polyangiitis, Hashimoto's thyroiditis, Graves' disease, idiopathic thrombocytopenic purpura, Addison's disease, rheumatoid arthritis (RA), ankylosing spondylitis, polymyositis (PM), dermatomyositis (DM) and multiple sclerosis (MS).

In one embodiment, if a nanostructure of the invention is for use in the prevention or treatment of an autoimmune disease, said first target cell is preferably an immune cell, e.g. macrophages and/or neutrophils. In one embodiment, the nanostructure is for use in a method of preventing or treating a disease, preferably for use in a method of specifically binding a first target cell, e.g. immune cell, to a second target cell, e.g. diseased cell. In one embodiment, a nanostructure of the invention allows to specifically and selectively bind at least two cell types to each other. In one embodiment, a method of preventing or treating a disease comprises a method of forming a synapse between a first target cell and a second target cell. In one embodiment, a method of preventing or treating a disease comprises a contacting a first target cell and a second target cell, e.g. to form a synapse such as an immunological synapse. In one embodiment, a method of preventing or treating a disease comprises providing a specific distance between a first target and a second target. In one embodiment, the nanostructure is for use in providing a specific distance between a first target and a second target, e.g. between a first target cell and a second cell or between a sensor and a target cell. In one embodiment, a nanostructure is for use in inducing, promoting, stabilizing, and/or inhibiting, preferably inducing and/or promoting, a formation of a synapse in vivo or in vitro.

The term "effective amount", as used herein, relates to an amount sufficient to evoke a desired effect, such as a therapeutic effect and/or a binding of a first target and a second target and/or the formation of a synapse. To calculate the amount required to be an "effective amount" such as a therapeutically effective amount, the skilled person and/or the physician can use data obtained from cell culture assays and animal studies to formulate a range of dosage for use in humans. The dosage of such nanostructures and/or compositions lies preferably within a range of circulating concentrations that include the ED50 with little or no toxicity. The dosage may vary within this range depending upon the dosage form employed and the route of administration utilized. For any compound used, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser, together with instructions for administration.

The term "drug", as used herein, relates to any medicament, such as a chemotherapeutic agent, immunosuppressant, and/or checkpoint inhibitor. In one embodiment, a chemotherapeutic agent is a cytotoxic agent typically used in the chemotherapy of cancer, or to a checkpoint inhibitor typically used in cancer immunotherapy.

In one embodiment, in the context of a method of the invention, the term "providing a nucleic acid nanostructure" comprises providing an assembled nucleic acid nanostructure and/or providing a building material for a nucleic acid nanostructure, such as a scaffolding strand and one or more staple strands. In one embodiment, a method of preparing a nanostructure of the invention comprises a step of allowing self-assembly of a nanostructure and purifying said self-assembled nanostructure, for example step i) of said method, i.e. a step of providing a nucleic acid nanostructure, comprises a step of allowing self-assembly and subsequent purification. In one embodiment, allowing self-assembly comprises mixing at least one scaffolding strand and one or more staple strands, optionally further comprises adjusting the ionic strength, e.g. by adding MgCl₂ to 20 mM, and/or further comprises using a temperature protocol running a sequence of temperatures. In one embodiment, said purifying comprises removing the remaining excess of staple strands, e.g. by a precipitation such as PEG-precipitation, filtration, and/or liquid chromatography. In one embodiment, self-assembly and/or a step of allowing self-assembly comprises a step of denaturation and a step of cooling. In one embodiment, a step of denaturation is performed at a temperature of from 50°C to 80°C, preferably 60°C to 70°C, e.g. about 65°C, for a period of from 1 minute to 45 minutes, preferably 10 to 20 minutes, e.g. about 15 minutes. In one embodiment, a step of cooling is performed at a temperature of from 0°C to 70°C, preferably 20°C to 60°C, e.g., about 50°C to 58°C. In a preferred embodiment, a step of cooling is performed as a gradual cooling, preferably at a temperature from about 58°C to about 50°C with a decrease of 1°C per hour. A person skilled in the art understands that the protocol for self-assembly depends on the design and/or nucleic acid sequence of the nanostructure, and that the protocol can be adjusted in line with known protocols for preparing nanostructures. In one embodiment, a nanostructure is configured to have a scaffolding sequence that does not comprise a sequence forming secondary structures. In one embodiment, a nucleic acid sequence conjugated to a targeting agent, preferably a nucleic acid sequence conjugated to a targeting agent which is complementary to a sequence of the nanostructure, is configured to consist of a sequence that does not form secondary structures. In one embodiment, the nucleic acid sequences of a nanostructure, e.g. the nucleic acid sequences of a scaffolding strand and staple strands, the nucleic acid sequence of a nucleic acid handle e.g. DNA handle, and/or the nucleic acid conjugated to a targeting agent are configured to not form a secondary structure. In one embodiment, a self-assembled nanostructure has one or more vacancies, i.e. single-stranded nucleic acid sequences that can be bound by complementary nucleic acid sequences conjugated to a targeting agent.

In one embodiment, a method of preparing a nanostructure comprises a step of coupling a targeting agent, preferably a first targeting agent, a second targeting agent, and/or a third targeting agent, to the nanostructure. In one embodiment, the terms "coupling", "binding to", "attaching to", and "conjugating" are used interchangeably. In one embodiment, the terms "coupled", "bound", "attached", and "conjugated" are used interchangeably. In one embodiment, the step of coupling a targeting agent to the nanostructure comprises providing a first targeting agent, a second targeting agent, and a third targeting agent, each having a unique nucleic acid sequence attached thereto, which is complementary to a nucleic acid strand of the nanostructure, e.g. a scaffolding strand of the nanostructure or a DNA handle strand attached to the nanostructure. In one embodiment, due to the complementarity of the nucleic acid sequence attached to the targeting agents to a nucleic acid sequence of the nanostructure, such targeting agents bind to the nanostructure via the complementary nucleic acid strands, preferably automatically bind to the nanostructure via the complementary nucleic acid strands. In one embodiment, when referring to a "unique" nucleic acid sequence, such term means that the first targeting agent, the second targeting agent, and the third targeting agent have different nucleic acid sequences attached thereto. Such "unique" nucleic acid sequences are configured to be complementary to a specific sequence and/or to bind to a specific location of the nanostructure. In one embodiment, a nucleic acid sequence conjugated to a first targeting agent is different from a nucleic acid sequence conjugated to a second targeting agent which is different from a nucleic acid sequence conjugated to a third targeting agent. In one embodiment, when referring to "targeting agents", the first targeting agent, the second targeting agent, and/or the third targeting agent is/are meant.

In one embodiment, the method of preparing a nanostructure comprises a step of mixing the nucleic acid nanostructure with a first targeting agent, second targeting agent, and/or third targeting agent, preferably a first targeting agent, second targeting agent, and third targeting agent, e.g. at equimolar ratios or with targeting agent excess, optionally, further comprising a step of incubating said mixed nucleic acid nanostructure and targeting agents. In one embodiment, the method of the invention comprises a step of removing excess targeting agents and/or unbound targeting agents after a step of mixing a nucleic acid nanostructure with targeting agents, e.g. by a precipitation such as PEG-precipitation, filtration, and/or liquid chromatography. In one embodiment, a step of incubating a nanostructure and targeting agents is performed at a temperature of from 20°C to 50°C, preferably of from 30°C to 40°C, e.g. about 37°C, for a period of from 1 minute to 3 hours, preferably 30 minutes to 1.5 hours, e.g. about 1 hour.

In one embodiment, a method of the invention comprises a step of obtaining a nucleic acid nanostructure comprising said first targeting agent, said second targeting agent, and said third targeting agent, wherein said obtaining comprises
a) mixing one or more building materials for a nanostructure, obtaining an assembled nanostructure, and subsequently contacting said assembled nanostructure with one or more targeting agents, thereby obtaining a nanostructure comprising targeting agents; and/or
b) mixing one or more building materials for nanostructure with one or more targeting agents and obtaining a nanostructure comprising targeting agents; and/or
c) mixing an assembled nanostructure with one or more targeting agents, thereby obtaining a nanostructure comprising targeting agents.

In one embodiment, a method of preparing a nanostructure, comprises the steps:
iv) providing a nucleic acid nanostructure, preferably rigid nucleic acid nanostructure,
   preferably, by allowing self-assembly of a nanostructure, e.g. mixing at least one scaffolding strand and one or more staple strands, and purifying said self-assembled nanostructure,
   wherein, optionally, said self-assembly comprises a step of denaturation and a step of cooling, preferably gradual cooling;
v) providing a first targeting agent, a second targeting agent, and a third targeting agent, wherein each of said targeting agents is conjugated to a nucleic acid strand that is complementary to a nucleic acid strand, e.g. scaffolding strand or DNA handle strand, of said nanostructure;
vi) preferably, mixing the nucleic acid nanostructure with a first targeting agent, second targeting agent, and/or third targeting agent, optionally incubation;
vii) optionally, removing unbound first targeting agent, second targeting agent, and/or third targeting agent;
viii) obtaining a nucleic acid nanostructure comprising said first targeting agent, said second targeting agent, and said third targeting agent.

In one embodiment, a method of preparing a nanostructure comprising targeting agents comprises a step of attaching a targeting agent-nucleic acid conjugate to the nanostructure, said method comprising the steps:
1. Allowing a nanostructure to self-assemble, and purification of said assembled nanostructure, e.g. DNA-origami object, preferably by mixing one or more scaffolding strand, preferably one scaffolding strand, with one or more staple strands,
   optionally adjusting an ionic strength (e.g. by adding MgCl₂ to 20 mM) and subjecting the mixture to a sequence of specific temperatures,
   optionally removing an excess of unbound staples strands (e.g. with PEG-precipitation, filtration, liquid chromatography);
2. Coupling a nucleic acid strand to a targeting agent, wherein each of a first targeting agent, a second targeting agent, and a third targeting agent are coupled to a different nucleic acid sequence, wherein said nucleic acid sequence is preferably complementary to a DNA sequence that protrudes from the nanostructure e.g. DNA-origami object, for example a nucleic acid handle, or to a sequence of a scaffold DNA strand;
3. Mixing of the nanostructures e.g. DNA-origami objects with the targeting agent-nucleic acid conjugates e.g. DNA-antibody conjugates,
   preferably at equimolar ratios or with targeting agent-nucleic acid conjugate, e.g. DNA-antibody-conjugate, excess,
   preferably incubating the nanostructure and the targeting agents at about 37°C for about 1h;
4. Removing excess targeting agent-nucleic acid conjugate e.g. DNA-antibody-conjugate, for example using PEG-precipitation, filtration, liquid chromatography.

In one embodiment, a nanostructure may be stabilized against nuclease degradation, against disassembly by low ionic-strength environments, and/or in-vivo conditions using coatings as described in ["Oligolysine-based coating protects DNA nanostructures from low-salt denaturation and nuclease degradation", Ponnuswamy et al., Nat Commun 2017; WO2015070080] and/or stabilization methods described in ["Sequence-programmable covalent bonding of designed DNA assemblies", T. Gerlin, Science Advances 2018; WO2019234122].
In one embodiment, a nanostructure of the invention, e.g. rigid single-domain nanostructure with one configuration, is highly advantageous because it can be stabilized using said stabilization methods without an impairment of its conditional cell-recruiting function.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. It will be appreciated, that the use of the singular article "a" or "the" within this document is not meant to limit the scope of the invention expect if specifically stated. That is, generally "a" may also refer to more than one. In other words, "a" can generally be read as "at least one". For example, "a targeting agent" also includes a plurality of targeting agent. In one embodiment, the term "as defined above" relates to "as defined in any of the embodiments above".

The terms "of the [present] invention", "in accordance with the invention", "according to the invention" and the like, as used herein, are intended to refer to all aspects and embodiments of the invention described and/or claimed herein

Whenever method steps are recited in this description and also in the appended claims, it should be noted that the order in which the steps are recited in this text may be accidental. That is, unless otherwise specified or unless clear to the skilled person, the order in which steps are recited may also differ. For example, when the present document states that, e.g., a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) can encompass the situation that step (X) is performed directly before step (Z), but also the situation that step (X) is performed before one or more steps, e.g. (Y1), (Y2), (Y3), followed by step (Z). Corresponding considerations apply when terms like "after" or "before" are used.

### BRIEF DESCRIPTION OF THE FIGURES

The present invention is now further described by reference to the following figures.

All methods mentioned in the figure descriptions below were carried out as described in detail in the examples.
**Figure** 1 shows an exemplary scheme of the mechanism of conditional T-cell mediated killing of target cells with a DNA-origami antibody platform.
   An exemplary nanostructure of the invention is shown, particularly an exemplary DNA-origami carrier platform with two targeting agents A1 (exemplary second targeting agent) and A2 (exemplary third targeting agent) against a target cell (e.g. antibodies against target-cell antigens) and one targeting agent B1 (exemplary first targeting agent) against an immune cell (e.g. antibody against an immune-cell antigen). The longitudinal axis is indicated by the letter 1, and the transverse axis is indicated by the letter t. **A**, The antibody platform can bind with both target-cell antibodies to cells that carry both antigens RA1 and RA2 (exemplary second target molecule and third target molecule). In order to bind with both antibodies A1 and A2 to their respective targets RA1 and RA2 the platform needs to adopt an orientation where the longitudinal axis is parallel to the target cell's cell surface. This presents the immune-cell antibody B1, which can bind to a first target molecule such as an immune-cell antigen (e.g. CD3 on a cytotoxic T cell). The platform's dimensions, the targeting agents e.g. antibodies, and the antibody attachment are chosen such that the distance between the cell surfaces d is smaller than the minimum distance dₘᵢₙ (e.g. 10 nm) required for immune-synapse formation. **B**, If a cell carries only antigen RA1, the antibody platform may bind with antibody A1, but it may not adopt a stable parallel orientation with respect to the cell surface. The platform will fluctuate and the antibody B1 will be not accessible for an immune cell. Furthermore, the antibody platform may dissociate. A low-affinity antibody may be used for the A1 position to facilitate quick dissociation. **C**, If a cell carries only antigen RA2, the antibody platform may bind with antibody A2, but it may not adopt a stable parallel orientation with respect to the cell surface. The antibody B2 will be accessible to recruit an immune cell. The distance d between the cell surfaces is however larger than the distance dₘᵢₙ allowed for immune-synapse formation. Cells carrying only RA2 will therefore not be killed by the recruited immune cells.
**Figure 2** shows schemes of exemplary nanostructures of the invention.
   **A**, Schemes of a rod-like DNA-origami carrier platform (2D scheme at the top, 3D schemes in the middle and too) with attached targeting agents against a target cell (A1, A2) and an immune cell (B1). 1 indicates the direction of the longitudinal axis, t1 and t2 indicate the directions of the transverse axes. **B**, As in A but with cylinders representing double-stranded interconnected DNA helices and Fab-Fragments as targeting agents. **D**, caDNAno design diagram of a rod-like antibody-DNA-origami carrier platform. Exemplary targeting-agent-attachment positions are indicated with black circles.
**Figure 3** shows schemes of exemplary nanostructures of the invention having a recess.
   **A**, **B**, **C**, Schemes of a rod-like antibody-DNA-origami platform with a recess and attached targeting agents against target cells (A1, A2) and against immune cells (B1). 1 indicates the direction of the longitudinal axis, t1 and t2 indicate the directions of the transverse axes. **A**, 2D schematic. **B**, **C**, Cylinders represent double-stranded DNA helices and Fab-Fragments as targeting agents. **D**, A recessed targeting agent B1 would reduce the accessibility of the antibody B1 in both cases where only one antigen RA1 or RA2 is present.
**Figure** 4 shows schematic representations of exemplary DNA-origami antibody platforms (grey cylinders represent DNA double helices) with attached Fab-Fragments (cyan, blue, purple, and orange) as targeting agents. Rod-like (A, B, C, D), plate-like (E), and L-shaped-like (F) platforms for conditional synapse formation. DNA-origami platforms may carry different numbers and valencies of first targeting agents (B1 and B2) against a first cell (e.g. immune cell). DNA-origami platforms may carry different numbers and valencies of second targeting agents (A1, A2, and A3) against a second cell (e.g. cancer cell) at different positions. 1 indicates the direction of the longitudinal axis, t1 and t2 indicate the directions of the transverse axes. Second targeting agents (A1, A2, and A3) are arranged on the DNA-origami platforms to promote a parallel orientation of the longitudinal axis to the second cell's cell surface if all second targeting agents have bound to their target molecules.

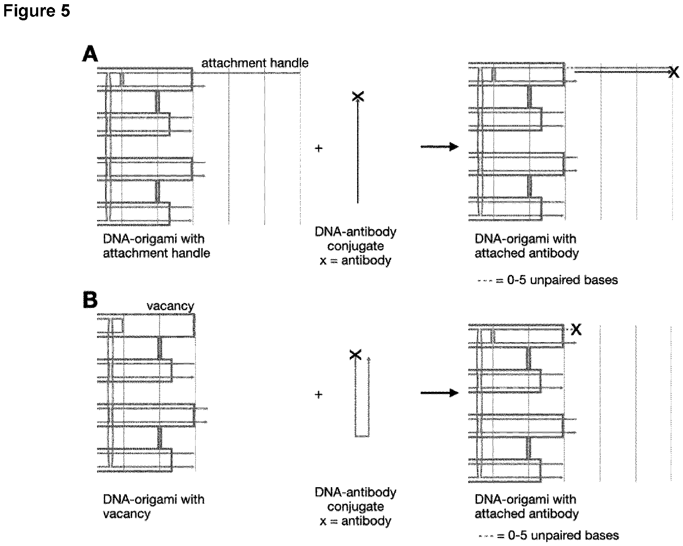

**Figure 5** shows an exemplary attachment strategy of exemplary nanostructures e.g. DNA-antibody conjugates. **A,** Part of a caDNAno diagram of a DNA-origami object with a protruding single-stranded DNA-attachment handle (left). The DNA sequence of the DNA-antibody conjugate (x is the position of the antibody) is complementary to the attachment handle's sequence. By mixing the DNA-origami object with the DNA-antibody conjugate (equimolar ratio, incubation for 1h at 37°C), the DNA handle on the antibody will hybridize to the DNA origami's attachment handle (right). The sequences may be chosen such that 0 to 5 bases (e.g. thymines) remain unpaired (dotted line in caDNAno diagram) to improve or restrict the antibody's range of spatial angles. **B**, Part of a caDNAno diagram of a DNA-origami object with an binding site (i.e. vacancy) for a single-stranded DNA oligonucleotide (left). The DNA sequence of the DNA-antibody conjugate (x is the position of the antibody) is complementary to the scaffold's sequence at the binding site. By mixing the DNA-origami object with the DNA-antibody conjugate (equimolar ratio, incubation for 1h at 37°C), the DNA handle on the antibody will hybridize to the DNA origami's vacancy (right). The sequences may be chosen such that 0 to 5 bases (e.g. thymines) remain unpaired (dotted line in caDNAno diagram) to improve or restrict the antibody's range of spatial angles.

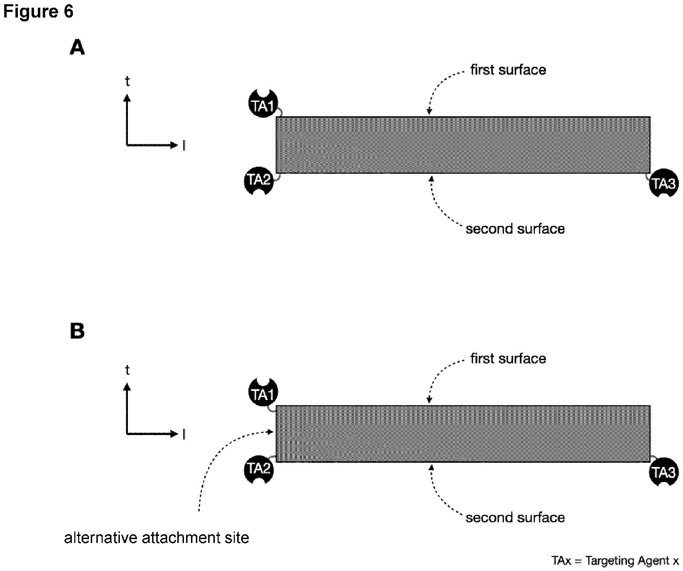

**Figure 6** shows two exemplary attachment positions of the targeting agents (TA1, TA2, and TA3). **A,** the first targeting agent (TA1) is directly attached at the first surface of the nanostructure e.g. the DNA-origami platform, and the second and third targeting agent (TA2 and TA3) are directly attached at the second surface. 1 indicates the direction of the longitudinal axis and t indicates the direction of the transverse axis. **B**, the first targeting agent (TA1) is attached at the adjacent surface of the first surface, e.g. a third surface or fourth surface, to the DNA-origami platform. For example, the attachment position can be close to the edge where the first and its adjacent surface, e.g. the third surface or fourth surface, meet. This attachment strategy allows the first targeting agent to bind to a first target, e.g. an immune cell, whose surface, e.g. cell surface, is parallel to the first surface while providing a smaller target-target distance, e.g. smaller cell-cell distance. The second and third targeting agent (TA2 and TA3) are attached at surfaces that are adjacent to the second surface, e.g. the third or fourth surface, at positions that are close to the edges. Attachment position shown in B may provide a smaller distance between the targeting agents' binding sites and a close attachment of the DNA-origami to the cell surfaces. Thus it provides a small distance between the first target and the second target, e.g. the first and second cell, while maintaining the targeting agents in a plane of the first or second surface, respectively. 1 indicates the direction of the longitudinal axis and t indicates the direction of the transverse axis.

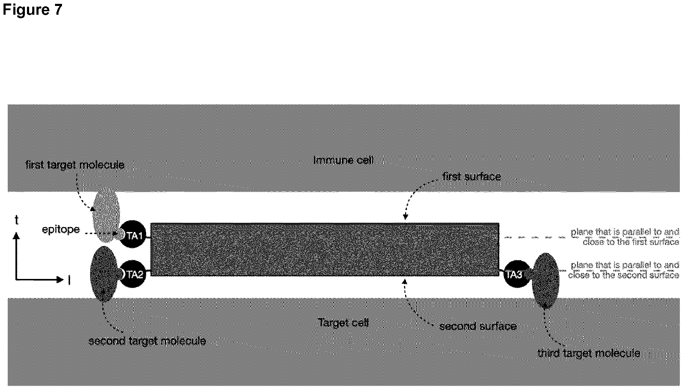

**Figure** 7 shows an exemplary nanostructure of the invention, e.g. a DNA-origami platform with attached targeting agents (TA1, TA2, TA3) that have bound to target molecule's epitopes (depicted as circular protrusions form the ellipse) of a first target, e.g. first target cell (top, immune cell) and a second target, e.g. second target cell (bottom, target cell). The targeting agents are attached in planes that are parallel to and close to the first and second surfaces of the DNA-origami platform, but not directly at the outer surfaces. This attachment strategy allows for an improved orientation of targeting agents when they are bound to the target molecule's epitopes. This attachment strategy thus allows for small target-to-target distances, e.g. small cell-to-cell distances, where the distance is only limited by the maximum transverse extension of the DNA-origami platform. 1 indicates the direction of the longitudinal axis and t indicates the direction of the transverse axis.

In the following, reference is made to the examples, which are given to illustrate, not to limit the present invention.

### EXAMPLES

### Example 1: Design and construction of multi-valent and multi-specific DNA-origami antibody objects.

DNA objects were designed and assembled according to the DNA-origami method [5-7]. Antibodies were attached to DNA objects by conjugating a DNA strand (e.g. 25 bases) to the antibody. Ion-exchange chromatography purified antibody-DNA conjugates were mixed with PEG-purified [8] DNA-origami objects at equimolar ratios or with antibody-DNA-conjugate excess. Single-stranded DNA handles were placed on the surface of the DNA origami objects at different positions. These protruding DNA handles serve as antibody-attachment sites and may be included by the designer in the folding reaction. Alternatively, the antibody was modified with a sequence that is complementary to the scaffold and thus directly binds into the DNA origami object. Finally, a DNA-origami-antibody construct is self-assembled by relying on DNA-DNA hybridization of the protruding strand or the scaffold strand with the complementary antibody-conjugated strand. DNA-origami-antibody objects may be further purified or stabilized against nucleases and low ionic-strength conditions according to established methods [9, 10].

### Example 2: Protocol for the attachment of antibody-DNA conjugates to DNA-origami objects

1. Self-Assembly and purification of the DNA-origami objects: Mixing scaffold and staple strands (at least one staple strand per scaffold strand), adjusting the ionic strength (e.g. by adding MgC12 to 20 mM) and subjecting the mixture to a sequence of specific temperatures. Finally, removing the remaining excess of staples strands (e.g. with PEG-precipitation, filtration, liquid chromatography).
2. Coupling of DNA strands to the targeting agents e.g. antibodies. Each targeting agent e.g. antibody type carries a DNA strand with a unique sequence. The DNA sequence is either complementary to a DNA sequence that protrudes from the DNA-origami object or to a sequence of the scaffold DNA strand.
3. Mixing of the DNA-origami objects with DNA-antibody conjugates (at equimolar ratios or with DNA-antibody-conjugate excess) and incubation at 37°C for 1h.
4. Removal of excess DNA-antibody-conjugate using e.g. with PEG-precipitation, filtration, liquid chromatography.

### REFERENCES

[1] "Induction of regular cytolytic T cell synapses by bispecific single-chain antibody constructs on MHC class I-negative tumor cells", Offner et al, Molecular Immunology 2005.
[2] "The immune synapse clears and excludes molecules above a size threshold", Cartwright et al., Nature Communications 2014, and "The immunological synapse", M. L. Dustin, Cancer Immunol Res. 2015.
[3] "Epitope distance to the target cell membrane and antigen size determine the potency of T cell-mediated lysis by BiTE antibodies specific for a large melanoma surface antigen", Bluemel et al., Cancer Immunol Immunother 2010.
[4] "Case Report of a Serious Adverse Event Following the Administration of T Cells Transduced With a Chimeric Antigen Receptor Recognizing ERBB2", R. A. Morgan et al., Mol Ther. 2010.
[5] "Folding DNA to create nanoscale shapes and patterns", P. Rothemund, Nature 2006.
[6] S. M. Douglas et al, "Self-assembly of DNA into nanoscale three-dimensional shapes", Nature 2009.
[7] "How we make DNA origami", K. Wagenbauer, ChemBioChem 2017.
[8] E. Stahl, T. G. Martin, F. Praetorius, H. Dietz, "Facile and scalable preparation of pure and dense DNA origami solutions", Angewandte Chemie 53, 12735-12740 (2014).
[9] "Oligolysine-based coating protects DNA nanostructures from low-salt denaturation and nuclease degradation", N. Ponnuswamy, Nat Commun 2017.
[10] "Sequence-programmable covalent bonding of designed DNA assemblies'" T. Gerling, Science Advances 2018.
[11] "Origins of the cytolytic synapse", Maike de la Roche, Yukako Asano and Gillian M. Griffith, Nat Rev Immunol, 2016 Jul, https://doi.org/10.1038/nri.2016.54.
[12] "Functional Anatomy of T Cell Activation and Synapse Formation", D. R. Fooksman et al., Annu. Rev. Immunol. 2010, https://doi.org/10.1146/annurev-immunol-030409-101308

The features of the present invention disclosed in the specification, the claims, and/or in the accompanying figures may, both separately and in any combination thereof, be material for realizing the invention in various forms thereof.

## Claims

1. A nucleic acid nanostructure comprising a first surface and a second surface, wherein said first surface and said second surface are located at opposing sides of said nanostructure, wherein said first surface comprises at least a first targeting agent and said second surface comprises at least a second targeting agent and at least a third targeting agent.

2. The nucleic acid nanostructure according to claim 1, wherein said nanostructure has a longitudinal axis and a transverse axis, wherein said first surface and said second surface are located at opposing sides along said longitudinal axis.

3. The nucleic acid nanostructure according to claim 1 or 2, wherein the nanostructure has a maximum longitudinal extension along said longitudinal axis which is larger than a maximum transverse extension along said transverse axis, preferably larger than a synaptic distance, more preferably larger than at least 10 nm, e.g. at least 25 nm; and/or
wherein the nanostructure has a maximum transverse extension along said transverse axis which is smaller than a maximum longitudinal extension along said longitudinal axis, preferably smaller than a synaptic distance, more preferably smaller than 25 nm, e.g. ≤ 10 nm.

4. The nucleic acid nanostructure according to any one of the foregoing claims, wherein the first targeting agent is configured to bind to a first target molecule on a first target, preferably first target cell, more preferably immune cell, wherein the second targeting agent is configured to bind to a second target molecule on a second target, preferably second target cell, more preferably diseased cell, and wherein the third targeting agent is configured to bind to a third target molecule on said second target, preferably second target cell, more preferably diseased cell.

5. The nucleic acid nanostructure according to any one of the foregoing claims, wherein the first targeting agent, the second targeting agent, and the third targeting agent are independently selected from an antibody or antigen-binding fragment thereof, an antigen-binding peptide, a Fab fragment, a F(ab')₂ fragment, a Fv fragment, a diabody, a single chain Fv fragment, a (scFv)₂, a tetrabody, a triabody, a disulfide bond-stabilized Fv (dsFv), a Fc domain, an engineered Fc domain, a DNA aptamer, a RNA aptamer, a peptide nucleic acid (PNA), a polypeptide, a peptide, a glycoprotein, a peptidomimetic, an anticalin, an Affilin, an Affimer, an Affitin, an Alphabody, a nanobody, DARPin, a receptor ligand, a receptor or fragment thereof, and a receptor domain,
preferably independently selected from an antibody or antigen-binding fragment thereof, an antigen-binding peptide, a Fab fragment, a F(ab')₂ fragment, a Fv fragment, a diabody, a single chain Fv fragment, a (scFv)₂, a tetrabody, a triabody, a disulfide bond-stabilized Fv (dsFv), a Fc domain, an engineered Fc domain, a polypeptide, a peptide, a glycoprotein, a peptidomimetic, an anticalin, an Affilin, an Affimer, an Affitin, an Alphabody, a nanobody, DARPin, a receptor ligand, a receptor or fragment thereof, and a receptor domain.

6. The nucleic acid nanostructure according to any one of the foregoing claims, wherein the second targeting agent and the third targeting agent are spaced at a distance from each other, preferably at a distance along the longitudinal axis larger than a synaptic distance, for example of at least 15 nm, preferably at least 20 nm, more preferably at least 40 nm; and/or
wherein said nanostructure is a rigid nanostructure, and/or is a nanostructure without a hinge region and/or is a nanostructure having only one configuration, and/or is a nanostructure comprising or being a DNA origami, wherein said DNA origami has only one configuration; and/or
wherein the nanostructure comprises a maximum length, and wherein the maximum length is smaller than 1000 nm, preferably smaller than 500 nm, such as 100 nm, wherein, preferably, said maximum length is a length of the maximum longitudinal extension.

7. The nucleic acid nanostructure according to any one of the foregoing claims,
wherein said first target cell is an immune cell, preferably selected from the group consisting of lymphocytes, e.g. T cells, cytotoxic T cells, B cells, natural killer cells, natural killer T cells, CAR-T cells, monocytes, macrophages, and neutrophils, more preferably selected from the group consisting of lymphocytes, e.g. T cells, cytotoxic T cells, B cells, natural killer cells, natural killer T cells, and CAR-T cells; and/or
wherein said second target cell is a diseased cell, preferably selected from a cancer cell, a tumor cell, a cell involved in an autoimmune response, and an infected cell, e.g. a cell infected with a virus, mycoplasma, bacterium, or parasite.

8. The nucleic acid nanostructure according to any one of the foregoing claims, wherein said first target molecule is a surface molecule of an immune cell, preferably selected from CD3, CD3δ/ε, CD3γ/ε, TCR, TCRα, TCRβ, CD2, CD5, CD28, OX40, 4-1BB, CD16, Ly49, NKp30 (CD337), NKp44 (CD336), NKp46 (NCRi), CD3ζ (CD247), CD27, CD40, CD137, CD64, CD89, toll-like receptors (TLR), cytokine receptors, a Fc domain, an engineered Fc domain, GITR, and ICOS; and/or
wherein said second target molecule and said third target molecule are independently selected from CD2, CD7, CD10, CD13, CD15, CD19, CD24, CD28, CD29, CD33, CD34, CD38, CD44, CD45, CD49f, CD56, CD57, CD60a, CD66/CEA, CD79a, CD117, CD123, CD138, CD140b, CD227/MUC1, CD243/MDR, CD244, CD326/EpCAM, CD340/HER2, VEGF-R, EGFR, CSPG4/MCSP, MAGs, CA125, PSMA, HLA-DR, carbonic anhydrase 9, aquaporin, PSMA, TIM3, CLLi/CLEC12A, EGFR v3, and HLA-A2, wherein, preferably, said second target molecule and said third target molecule are not identical; and/or
wherein said second target molecule and said third target molecule are a combination of target molecules, wherein said combination is selected from CD33 and CD123, CD326/EpCAM and CD10, CD326/EpCAM and CD340/HER2, CD326/EpCAM and VEGF-R, CD326/EpCAM and EGFR, CD326/EpCAM and CD243/MDR, CSPG4/MCSP and CD326/EpCAM, CSPG4/MCSP and MAGs, CA125 and CD227/MUC1, CA125 and CD227/MUC1, CD56 and CD140b, CD56 and CD60a, EGFR and CD340/HER2, PSMA and CD340/HER2, CD15 and EGFR, CD44 and CD117, CD44 and CD326/EpCAM, CD34 and CD19, CD34 and CD79a, CD34 and CD2, CD34 and CD7, CD34 and HLA-DR, CD34 and CD13, CD34 and CD117, CD34 and CD33, CD34 and CD15, CD33 and CD19, CD33 and CD79a, CD33 and CD2, CD33 and CD7, CD33 and HLA-DR, CD33 and CD13, CD33 and CD117, CD33 and CD15, CD227/MUC1 and CD10, CD227/MUC1 and CD66/CEA, CD227/MUC1 and CD57, CD38 and CD138, CD24 and CD29, CD24 and CD49f, carbonic anhydrase 9 and aquaporin, CD19 and CD33, CD19 and CD22, CD28 and PSMA, CD227/MUC1 and EGFR, CD33 and TIM3, CD123 and TIM3, CLL1/CLEC12A and TIM3, CD244 and TIM3, CD33 and EGFR v3, CLL1/CLEC12A and EGFR v3, CD123 and EGFR v3, and CD45 and HLA-A2.

9. The nucleic acid nanostructure according to any one of the foregoing claims, wherein said nucleic acid comprises or consists of DNA; and/or
wherein the nanostructure comprises or is a DNA origami structure,
wherein the DNA origami structure comprises at least one scaffolding strand,
wherein the DNA origami structure further comprises a plurality of single-stranded oligonucleotide staple strands,
wherein each staple strand is at least partially complementary to at least one scaffolding strand, and
wherein each of the staple strands is configured to bind to at least one of the at least one scaffolding strand in at least one place, preferably in two or more distinct places, wherein the at least one scaffolding strand is folded and/or arranged such that the desired nanostructure is formed.

10. The nucleic acid nanostructure according to any one of the foregoing claims, wherein said nanostructure comprises a recess, preferably a recess oriented perpendicular to said maximum longitudinal extension,
wherein, preferably, said first targeting agent is bound to said nanostructure via said recess and/or at least a portion of said first targeting agent is located within said recess.

11. The nucleic acid nanostructure according to any one of the foregoing claims, wherein said nanostructure further comprises an active agent, preferably drug, and/or a marker, e.g. imaging marker, wherein said active agent and/or marker is/are coupled to said nanostructure, optionally via a linker.

12. A composition, preferably pharmaceutical composition, comprising a nucleic acid nanostructure of any one of claims 1-11, optionally further comprising a pharmaceutically acceptable excipient.

13. The nanostructure of any one of claims 1-11 or the composition of claim 12 for use in medicine.

14. The nanostructure of any one of claims 1-11 or the composition of claim 12 for use in a method of preventing or treating a disease selected from proliferative diseases, such as cancer, immunological disorders, e.g. autoimmune diseases, infectious disorders, e.g. viral diseases such as a human immunodeficiency virus (HIV) infection, metabolic disorders, and/or diabetes;
said method preferably comprising binding a first target cell and a second target cell using said nanostructure,
more preferably comprising inducing, promoting, stabilizing, and/or inhibiting a formation of a synapse, preferably only if the first, second, and third targeting agents have bound to their respective target molecules,
even more preferably comprising binding said first target cell and said second target cell using said nanostructure such that, as a result of said binding, said first target cell and said second target cell have a distance ≤ 25 nm, preferably ≤ 15 nm.

15. A method of preparing a nanostructure of any one of claims 1-11, comprising the steps:
i) providing a nucleic acid nanostructure, preferably rigid nucleic acid nanostructure,
ii) providing a first targeting agent, a second targeting agent, and a third targeting agent, wherein each of said targeting agents is conjugated to a nucleic acid strand that is complementary to a nucleic acid strand, e.g. scaffolding strand or DNA handle strand, of said nanostructure,
iii) obtaining a nucleic acid nanostructure comprising said first targeting agent, said second targeting agent, and said third targeting agent, preferably by self-assembly of said nanostructure.

16. Use of a nanostructure of any one of claims 1-11 or a composition of claim 12 for binding a first target and a second target;
preferably for inducing, promoting, stabilizing, and/or inhibiting a formation of a synapse between a first target cell and a second target cell, preferably only if the first, second, and third targeting agents have bound to their respective target molecules; more preferably comprising binding said first target, e.g. first target cell, and said second target, e.g. second target cell, using said nanostructure such that, as a result of said binding, said first target and said second target have a distance ≤ 25 nm, preferably ≤ 15 nm.
